# EUROPÄISCHE PATENTSCHRIFT

(11) **EP 0 512 661 B1**
(45) Veröffentlichungstag und Bekanntmachung des Hinweises auf die Patenterteilung: **14.01.1998**
(21) Anmeldenummer: 92250110.1
(22) Anmeldetag: 07.05.1992
(51) Int. Cl.: A61K 49/00, A61K 49/04, A61K 51/00

(54) **Makrocyclische Polymer-Komplexbildner, deren Komplexe, Verfahren zu ihrer Herstellung und diese enthaltende pharmazeutische Mittel**
Macrocyclic polymer-complexing, their complexes, method for their production and pharmaceutical agents containing them
Polymère-complexants macrocycliques, leur complexes, procédé pour leur préparation et agents pharmaceutiques les contenant

(30) Priorität: 10.05.1991 DE 4115789
(43) Veröffentlichungstag der Anmeldung: 11.11.1992
(73) Patentinhaber: SCHERING AKTIENGESELLSCHAFT, 13353 Berlin (DE)
(72) Erfinder: Schmitt-Willich, Heribert, Dr., W-1000 Berlin 45 (DE); Platzek, Johannes, Dr., W-1000 Berlin 33 (DE); Gries, Heinz, Dr., W-1000 Berlin 31 (DE); Schuhmann-Giampieri, Gabriele, Dr., W-1000 Berlin 45 (DE); Frenzel, Thomas, Dr., W-1000 Berlin 46 (DE)

(56) Entgegenhaltungen:
- EP-A- 0 186 947
- EP-A- 0 233 619
- EP-A- 0 255 471
- EP-A- 0 292 689
- EP-A- 0 414 288
- EP-A- 0 454 078
- EP-A- 0 457 438
- WO-A-91/14186
- BIOCONJUGATE CHEMISTRY, Band 1, Nr. 1, Januar/Februar 1990, Seiten 65-71, American Chemical Society, Washington, DC, US; P.F. SIEVING et al.: "Preparation and characterization of paramagnetic polychelates and their protein conjugates"

## Beschreibung

Die Erfindung betrifft den in den Patentansprüchen gekennzeichneten Gegenstand, das heißt neue makrocyclische Polymer-Komplexbildner und -Komplexe, diese Verbindungen enthaltende Mittel, die Verwendung der Komplexe in der Diagnostik und Therapie sowie Verfahren zur Herstellung dieser Verbindungen und Mittel.

Magnevist (GdDTPA/dimeglumin) ist das erste registrierte Kontrastmittel für die Kernspintomographie (MRI = Magnetic Resonance Imaging). Es ist besonders gut für die Diagnose pathologischer Bereiche (z. B. Entzündungen, Tumore etc.) geeignet. Die Verbindung wird nach intravenöser Injektion über die Nieren eliminiert; eine extrarenale Ausscheidung wird praktisch nicht beobachtet.

Ein Nachteil von Magnevist ist, daß es sich nach intravenöser Applikation gleichmäßig zwischen dem vasalen und dem interstitiellen Raum verteilt. Somit ist eine Abgrenzung der Gefäße gegenüber dem umliegenden interstitiellen Raum bei Anwendung von Magnevist nicht möglich.

Besonders für die Darstellung von Gefäßen wäre ein Kontrastmittel wünschenswert, das sich ausschließlich im vasalen Raum (Gefäßraum) verteilt. Ein solches blood-pool-agent soll es ermöglichen, mit Hilfe der Kernspintomographie gut durchblutetes von schlecht durchblutetem Gewebe abzugrenzen und somit eine Ischämie zu diagnostizieren. Auch infarziertes Gewebe ließe sich aufgrund seiner Anämie vom umliegenden gesunden oder ischämischen Gewebe abgrenzen, wenn ein vasales Kontrastmittel angewandt wird. Dies ist von besonderer Bedeutung, wenn es z.B. darum geht, einen Herzinfarkt von einer Ischämie zu unterscheiden.

Es besteht daher ein Bedarf an Kontrastmitteln, die den vasalen Raum markieren können (blood-pool-agent). Diese Verbindungen sollen sich durch eine gute Verträglichkeit und durch eine hohe Wirksamkeit (hohe Steigerung der Signalintensität beim MRI) auszeichnen.
Makromoleküle sind generell als Kontrastmittel für die Angiographie geeignet. Albumin-GdDTPA (Radiology 1987, 162: 205) z. B. zeigt jedoch 24 Stunden nach intravenöser Injektion bei der Ratte eine Anreicherung im Lebergewebe, die fast 30 % der Dosis ausmacht. Außerdem werden in 24 Stunden nur 20 % der Dosis eliminiert.

Das Makromolekül Polylysin-GdDTPA (Europäische Patentanmeldung, Publikations-Nr. 0 233 619) erwies sich geeignet als blood-pool-agent. Es zeigt jedoch den Nachteil, daß der offenkettige Komplexbildner DTPA bei erwünschten längeren Verweilzeiten des Kontrastmittels im Körper die Metallionen nicht fest genug bindet. Dies gilt auch für die in der Europäischen Patentanmeldung 0 186 947 beanspruchten Kohlenhydrat-Komplexe, sowie für die in J. Med. Chem. 1974, Vol. 17, 1304 beschriebenen mit Radioisotopen markierten Makromoleküle.

Neuerdings sind in der PCT WO 90/12050 Amide makrocyclischer Komplexbildner für die Bindung an Makromoleküle vorgeschlagen worden. Bekanntlich wird aber durch Umwandlung einer der Carboxygruppen der 1,4,7,10-Tetraazacyclododecan-tetraessigsäure (DOTA) in eine Amidgruppe die Stabilitätskonstante des Gadolinium-Komplexes um vier Größenordnungen herabgesetzt (A. D. Sherry et al., Inorg. Chem. 1989, 28, 620), sodaß das Problem der Abspaltung toxischer Metallionen aus den makromolekularen Komplexen auf diese Weise noch nicht befriedigend gelöst werden konnte.

Es bestand daher weiterhin die Aufgabe, Makromolekül-Komplexe zur Verfügung zu stellen, die sich intravasal verteilen bzw. spezifisch anreichem und gleichzeitig gegenüber den bekannten Strukturen eine hohe Stabilität zeigen. Diese Aufgabe wird durch die vorliegende Erfindung gelöst.
Es wurde nun gefunden, daß Makromolekül-Komplexe, in denen ein makrocyclischer Komplexbildner nicht über eine Amidbindung, sondern über eine ß-Hydroxyalkylgruppe gebunden ist, diese gewünschten Eigenschaften überraschenderweise zeigen.

Die Erfindung betrifft somit Polymerverbindungen der allgemeinen Formel I

(M)ₙA (I),

worin
- M: für den Rest eines makrocyclischen Komplexbildners,
- A: für ein Backbone-Molekül, das ein Defizit von n Amino-, n Hydroxy- oder n Carboxygruppen aufweist,
- n: für die Zahlen 1 bis 400,
dadurch gekennzeichnet, daß
- M: unabhängig voneinander für Komplexbildner der allgemeinen Formel IA steht 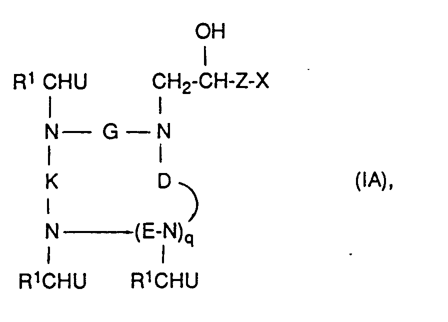 wobei
- D, E, G, K,: die gleich oder verschieden sein können, jeweils für die Gruppe -(CH₂)ₒ- mit o in der Bedeutung der Ziffern 2, 3, 4 oder 5
q für die Ziffern 0, 1 oder 2,
- R¹: für ein Wasserstoffatom, eine verzweigte oder unverzweigte C₁-C₄-Alkyl-, Phenyl-, C₇-C₁₂-Aralkylgruppe,
- U: für eine CO₂H- oder PO₃H₂-Gruppe,
- Z: für eine gegebenenfalls Imino-, Phenylen-, Phenylenoxy-, Phenylenimino-, Amid-, Hydrazid-, Carbonyl-, Estergruppe(n), Sauerstoff-, Schwefel- und/oder Stickstoff-Atom(e) enthaltende, gegebenenfalls durch Hydroxy-, Mercapto-, Oxo-, Thioxo-, Carboxy-, Ester- und/oder Aminogruppe(n) substituierte geradkettige, verzweigte, gesättigte oder ungesättigte C₁-C₂₀-Alkylengruppe,
- X: für eine -CONH-, -NHCO-, -NHCSNH-, -NHCONH-, -CO₂-, -O-, -OCO-, -COCH₂O-, -NR²CH₂CONH-, -NHCS-, -NR²CH₂COO-, -OCHR²CONH-,-OCHR²COO-, wobei R² für ein Wasserstoffatom oder die Gruppe -(CH₂)_{P}COOH mit p in der Bedeutung der Ziffern 1-5 steht,und für eine -NR-gruppe, worin R ein Wasserstoffatom, einen gegebenenfalls durch 1 bis 3 Carboxy-, 1 bis 3 Sulfonsäuren, 1 bis 5 Hydroxy-, 1 bis 5 C₁-C₁₀-Alkoxy-, 1 bis 5 C₆-C₁₀-Aryloxy-, 1 bis 5 C₇-C₁₁-Aralkoxy-, 1 bis 5 Ester-, 1 bis 5 Amidgruppen substituierten und/oder gegebenenfalls 1 bis 3 Carbonyl-, 1 bis 3 Ester-, 1 bis 3 Amid-, eine Sulfonylgruppe, 1 bis 10 Sauerstoff-, 1 bis 4 Stickstoffatome enthaltenden C₁-C₂₀-Kohlenwasserstoffrest oder ein zweites Molekül IA, das mit dem ersten nicht identisch sein muß, bedeutet, stehen, deren vollständig oder unvollständig metallierte Komplexe mit den Elementen der Ordnungszahlen 21-29, 31, 32, 37-39, 42-44, 49, 57-83 sowie deren Salze.

Die erfindungsgemäßen Komplexe sind aufgrund ihrer signalgebenden Eigenschaften und ihrer spezifischen Bindung an Organe, Gewebe, Tumoren und Zellen in vivo insbesondere zur Unterscheidung von gesundem und krankem Gewebe bei der Bildgebung mit Hilfe der Kemspintomographie, Röntgendiagnostik und Szintigraphie geeignet. Die erfindungsgemäßen Komplexbildner sind zur Entgiftung toxischer Metallionen geeignet und dienen als Träger von Metallionen, auch von Radioisotopen für die strahlentherapeutische und diagnostische Anwendung in der Nuklearmedizin.

Für die Kernresonanz- und die Röntgendiagnostik sind Komplexe geeignet, die Ionen eines Elements der Ordnungszahlen 21 bis 29, 42, 44 oder 57 bis 83, sowie gegebenenfalls Kationen anorganischer und/oder organischer Basen, Aminosäuren oder Aminosäureamide enthalten. Die für die Bildgebung erforderliche hohe Anzahl von Metallionen wird durch Kopplung einer Vielzahl makrocyclischer Komplexbildner oder Komplexe an Backbone-Moleküle erreicht.
Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21-29, 42, 44 und 58-70. Geeignete Ionen sind beispielsweise das Chrom(III)-, Mangan(II)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-Holmium(III)-, Erbium(III)- und Eisen(III)-ion.

Ist das erfindungsgemäße Mittel zur Anwendung in der Röntgendiagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 39, 42, 44, 57-83 enthalten, geeignet sind; dies sind beispielsweise das Lanthan(III)-ion und die oben genannten Ionen der Lanthanidenreihe.

Für die Radiodiagnostik und die Radiotherapie sind Komplexe geeignet, die als Zentralion ein Radioisotop der Elemente 27, 29, 31, 32, 37-39, 43, 49, 62, 64, 70, 75 oder 77 enthalten. Hier ist für die Bildgebung eine relativ geringe Anzahl von Metallionen und damit die Kopplung weniger makrocyclischer Komplexbildner erforderlich.
Auch eine Komplexierung mit unterschiedlichen Ionen ist wünschenswert. So kann beispielsweise ein partiell mit Yttrium-90 markierter polymerer Gadolinium-Komplex gleichzeitig zur Radio-Therapie wie auch zur NMR-diagnostischen Kontrolle der Anreicherung benutzt werden.
Auf diese Weise gelingt es, Diagnostik und Therapie mit Hilfe der erfindungsgemäßen Komplexkonjugate zu kombinieren.

Als Backbone-Moleküle, an die der Makrocyclus M gebunden ist, sind alle Polymere geeignet, die eine Vielzahl von Amino-, Carboxy- und/oder Hydroxy-Gruppen enthalten.

Beispielhaft genannt seien Polypeptide, (wie z. B. Polylysin, Polyornithin, Polyarginin, Polyasparaginsäure, Polyglutaminsäure, Polyserin), Polyallylamin, Poly[N-(2-aminoethyl)]methacrylamide und Polyaminkohlenhydrate (wie z. B. Polyaminodextran und Chitosane). Bevorzugt ist Polylysin; besonders bevorzugt ist Poly-L-Lysin.

Ist das erfindungsgemäße Mittel zur Anwendung in der NMR- und Röntgendiagnostik bestimmt, so steht n für die Zahlen 10 bis 400, bevorzugt für die Zahlen 10-200. Ist das erfindungsgemäße Mittel zur Anwendung in der Radiodiagnostik und Radiotherapie bestimmt, so steht n für Zahlen 1 bis 10.

Die in dem Makrocyklus M enthaltenden Brückenglieder D, E, G, K, die gleich oder verschieden sein können, bestehen aus 2 bis 5 CH₂-Gruppen, wobei Verbindungen mit o in der Bedeutung der Ziffern 2 und/oder 3 bevorzugt sind. Als bevorzugte Komplexbildner-Reste seien diejenigen der 1,4,7,10-Tetraazacyclododecan-1,4,7-triessigsäure, 1,4,7-Triazacyclononan-1,4-diessigsäure, 1,4,8,11-Tetraazatetradecan-1,4,8-triessigsäure, 1,5,9-Triazacyclododecan-1,5-diessigsäure genannt.
Als bevorzugte Substituenten R¹ seien das Wasserstoffatom, die Methyl-, die Ethyl-und Benzylgruppe genannt. Die für Z bzw. R stehende Alkylengruppe bzw. Alkylgruppe enthält 1-20 C-Atome und kann verzweigt, unverzweigt, cyclisch, gesättigt oder ungesättigt sein. Die für Z stehende Alkylengruppe kann durch 1-3, bevorzugt 1 Imino-, Phenylen-, Phenylenoxy-, Phenylenimino-und/oder 1-5, bevorzugt 1-3 Amid-, Hydrazid-, Carbonylgruppen und/oder 1-7, bevorzugt 1-5 Estergruppen, Sauerstoff-, Schwefel- und/ oder Stickstoff-Atom(e) unterbrochen sein und/oder 1-5, bevorzugt 1-3 Hydroxy-, Mercapto-, Oxo-, Thioxo-, Carboxy-, Ester- und/oder Aminogruppen tragen. Als bevorzugte Reste seien beispielhaft genannt:
-CH₂-O-C₆H₄-CH₂-CH₂-
-CH₂-O-CH₂-CH₂-O-CH₂-CH₂-
-CH₂-O-C₆H₄-
-CH₂-O-CH₂-CH(OH)-CH₂-O-CH₂-CH₂-
-CH₂-O-CH₂-CH(COOH)-CH₂-O-CH₂-CH₂- 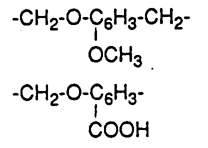
-CH₂-O-C₆H₄-O-CH₂-CH₂-
-CH₂-O-CH₂-CH₂-
-CH₂- , -(CH₂)₂- , -(CH₂)₃- , -(CH₂)₄-
-CH₂-O-C₆H₄-O-CH₂-CH₂-O-CH₂-CH₂- 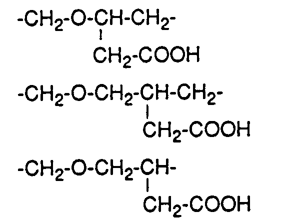
-CH₂-O-CH₂-CH₂-O-CH₂-CH₂-O-CH₂-CH₂-
-CH₂-O-CH₂-C₆H₄-CH₂-
-CH₂-O-C₆H₄-CH₂-CH₂-O-CH₂-CH₂-
-CH₂-O-C₆H₄-CH₂-O-CH₂-CH₂-
-C₆H₄-
-C₆H₄-O-CH₂-CH₂-
-C₆H₄-CH₂-

Als Reste R seien beispielhaft genannt:
-CH₂COOH; -(CH₂)₂COOH; -CH(COOH)CH₂COOH; -CH₂-CH(COOH)CH₂OH;
-CH₂SO₃H; -(CH₂)₂SO₃H; -COCH₃; -COCH₂OH; -COCHOHCH₂OH; -COCH₂O-CH₂COOH;
-CO(CHOH)₄CH₂OH; -COCH₂COOH; -CO(CH₂)₂COOH; -CO(CH₂)₃COOH;
-CO(CH₂)₄COOH;
-COCHOHCOOH; -CO(CHOH)₂COOH; -COCH₂CHOHCH₂COOH; -SO₂CH₂COOH; -SO₂(CH₂)₂COOH;
-SO₂CH₃;
-CO-CH₂-CH₂-CO-NH-CH₂-CONH-CH₂-COOH
-CO-CH₂-CH₂-CO-O-C₂H₅
-CO-CH₂-CH₂COO-CH₂-C₆H₅
-CO-CH₂-O-CH₂-CH₂-O-CH₂-CH₂-OCH₃
-CO-CH₂-C₆H₄-O-CH₂-CH₂-O-CH₂-CH₂-OCH₃
-CO-CH₂-CH₂-CONH-C₆H₄-O-CO-CH₃
-CO-CH₂-O-CO-C₇H₁₅
-CO-C₆H₄-O-CO-C₅H₁₁
-CO-CH₂-CH₂ - (CH₂)₈-OH
-CO-CH₂-CH₂-CONH-CH(CH₃)-COOH
-COCH₂CH₂-COO-CH(CH₃)-CH₂-NHCO-CH₂OH 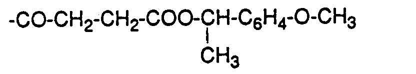
-SO₂-CH₂-CH₂-CH₂-CH₂-CH₂-CH₃
-SO₂-C₆H₄-COOH
-CO-CH₂-O-CH₂-CH₂-O-CH₂-CH₂-OCH₃
-CO-CH₂-NH-CO-CH₂-O-CH₂-CH₂-OCH₃
-CO-C₆H₄-COO-Et
-CO-CH₂-CH₂-COO-CH₂-CH₂-OCO-CH₂-CH₂-CONH-CH₂-CONH-C₆H₅

Die im Polymer-Komplex gegebenen vorhandenen restlichen aciden Wasserstoffatome können gegebenenfalls ganz oder teilweise durch Kationen anorganischer und/oder organischer Basen,

Aminosäuren oder Aminosäureamiden ersetzt sein. Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren wie z. B. Lysinmethylamid, Glycinethylamid und Serinmethylamid.

Die erfindungsgemäßen Verbindungen weisen die eingangs geschilderten gewünschten Eigenschaffen auf. Sie enthalten die für ihre Verwendung benötigte große Anzahl von Metallionen im Komplex stabil gebunden. Sie verteilen sich nur im vasalen Raum und können daher diesen mit Hilfe der Kernspintomographie markieren.
Der für Nebenwirkungen wie Schmerzen, Schädigungen der Blutgefäße und Herz-Kreislauf-Störungen verantwortliche Wert der Osmolalität ist im Vergleich zu Magnevist deutlich verringert.
Überraschend groß ist der Wert für die ein Maß der Bildgebung bei MRI darstellende Größe der Relaxivity; die Signalverstärkung ließ sich z. B. im Falle der Verbindung des Beispiels 4c gegenüber Magnevist um das 4fache steigern.

Im Vergleich zu den makromolekularen Kontrastmitteln auf der Basis von Kohlenhydraten, z. B. Dextran (Europäische Patentanmeldung, Publikations-Nr. 0 326 226), die - wie erwähnt - in der Regel nur 4,6 % des signalverstärkenden paramagnetischen Kations tragen, weisen die erfindungsgemäßen Polymer-Komplexe einen Gehalt von über 15 % des paramagnetischen Kations auf. Somit bewirken die erfindungsgemäßen Makromoleküle pro Molekül eine sehr viel höhere Signalverstärkung, was gleichzeitig dazu führt, daß die zur Kernspintomographie notwendige Dosis gegenüber der makromolekularer Kontrastmittel auf Kohlenhydratbasis erheblich kleiner ist.

Die erfindungsgemäßen Komplexe dienen als Kontrastmittel zur Darstellung der Gefäße mittels der Kernspintomographie. Es ist damit möglich, ischämisches Gewebe von normalem Gewebe zu unterscheiden. Aber auch andere Schädigungen der Blut-Gewebe-Schranke sind mit diesen Verbindungen zu erkennen. Bei Entzündungen und Tumoren im Gehirn ist die Blut-Hirn-Schranke so geschädigt, daß das Kontrastmittel das kranke Gewebe infiltrieren kann und somit das kranke Gewebe in der Kernspintomographie erkennbar wird. Aufgrund der Undurchlässigkeit der intakten Blut-Hirn-Schranke auch für kleine, aber hydrophile Moleküle konnte man Entzündungen und Tumore auch schon mit der niedermolekularen Verbindung Magnevist erkennen. Wendet man in diesen Fällen aber die erfindungsgemäßen Komplexe an, kann man aus zwei Gründen die Dosis um das 16fache reduzieren: 1. sie haben eine 4fach höhere Signalverstärkung und 2. verteilen sie sich in einem 4fach kleineren Raum, nämlich nur im Vasalraum, d. h. um gleiche Konzentrationen im Blut zu erreichen, genügt ein Viertel der Dosis.

Ein weiterer Vorteil der vorliegenden Erfindung liegt darin, daß nunmehr makrocyclische Komplexe unterschiedlichen Molekulargewichtes mit hydrophilen oder lipophilen Eigenschaften zugänglich geworden sind. Dadurch ist die Möglichkeit gegeben, Verträglichkeit und Pharmakokinetik dieser Polymer-Komplexe durch chemische Substitution zu steuern.

Die Herstellung der erfindungsgemäßen Polymer-Verbindungen erfolgt dadurch, daß man in an sich bekannter Weise n Moleküle der allgemeinen
Formel IA' 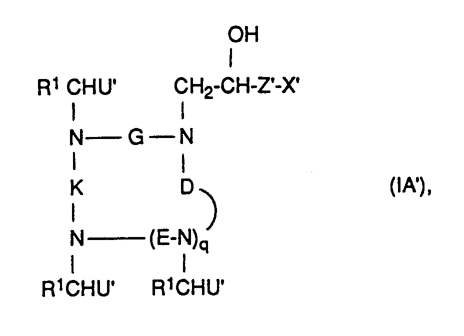 worin
- Z': für Z oder eine direkte Bindung steht,
- U': für eine CO₂Y oder PO₃HY-Gruppe mit Y in der Bedeutung eins Wasserstoffatoms, eines Metallionenäquivalents eines Elements der Ordnungszahlen 21-29, 31, 32, 37-39, 42-44 oder 57-83 oder einer Säureschutzgruppe,
- X': für eine C*O-, NH₂-, NCS-, NCO-, OFu-, Hal-, 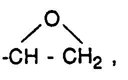 COCH₂Hal-, CHO- und Anhydridgruppe mit C*O in der Bedeutung einer aktivierten Carbonylgruppe, Fu in der Bedeutung einer Fluchtgruppe oder eines Wasserstoffatoms und Hal in der Bedeutung eines Fluor-, Chlor-, Brom- oder Jodatoms steht,
mit einem Backbone-Molekül A', das mindestens n Amino-, n Hydroxy- oder n-Carboxygruppen enthält, umsetzt,
anschließend gegebenenfalls reduziert und gewünschtenfalls mit einem den Substituenten R einführenden Reaktionspartner umsetzt, dann gegebenenfalls die Säureschutzgruppen entfernt und gegebenenfalls - in an sich bekannter Weise - mit mindestens einem Metalloxid oder Metallsalz eines Elements der Ordnungszahlen 21-29, 31, 32, 37-39, 42-44 oder 57-83 umsetzt (wobei die Metallkomplexierung auch vor der Einführung des Substituenten R erfolgen kann), und gegebenenfalls anschließend noch vorhandene acide Wasserstoffatome ganz- oder teilweise durch Kationen anorganischer und/oder organischer Basen, Aminosäuren oder Aminosäureamide substituiert.

Als Beispiel für eine aktivierte Carbonylgruppe -C*O- in Verbindungen der allgemeinen Formel IA' seien Anhydrid, p-Nitrophenylester, N-Hydroxy-Succinimidester, Säurechlorid und eine durch ein Carbodiimid-Derivat in situ aktivierte Carbonsäure, als Beispiel für eine Fluchtgruppe Cl, Br, I, CH₃-C₆H₄-SO₃, CH₃SO₃ und CF₃SO₃ genannt.

Als Säureschutzgruppen kommen niedere Alkyl-, Aryl- und Aralkylgruppen, beispielsweise die Methyl-, Ethyl-, Propyl-, n-Butyl-, t-Butyl-, Phenyl-, Benzyl-, Diphenylmethyl-, Triphenylmethyl-, bis(p-Nitrophenyl)-methylgruppe, sowie Trialkylsilylgruppen infrage.

Die Abspaltung der Säureschutzgruppen erfolgt nach den dem Fachmann bekannten Verfahren, beispielsweise durch Hydrolyse, Hydrogenolyse, alkalische Verseifung der Ester mit Alkali in wäßrigalkoholischer Lösung bei Temperaturen von 0 bis 50°C, saure Verseifung mit Mineralsäuren oder im Fall von z. B.tert.-Butylestern mit Hilfe von Trifluoressigsäure.

Die Synthese der gewünschten Polymer-Verbindungen erfolgt durch Umsetzung von Edukten der allgemeinen Formel IA', die Flucht-, aktivierte Carbonyl-, Amino-, Anhydrid-, Isocyanat-, Isothiocyanat- oder Aldehyd-gruppen enthalten, mit den Amino-oder Carboxy- oder Hydroxy-Gruppen der gewünschten Backbone-Moleküle nach literaturbekannten Standardmethoden, wobei im Falle der Umsetzung von Aldehyd- mit Amino-gruppen eine nachfolgende Reduktion durchgeführt werden muß.

Als ausgewählte Beispiele für Z'-X'-Reste seien genannt:

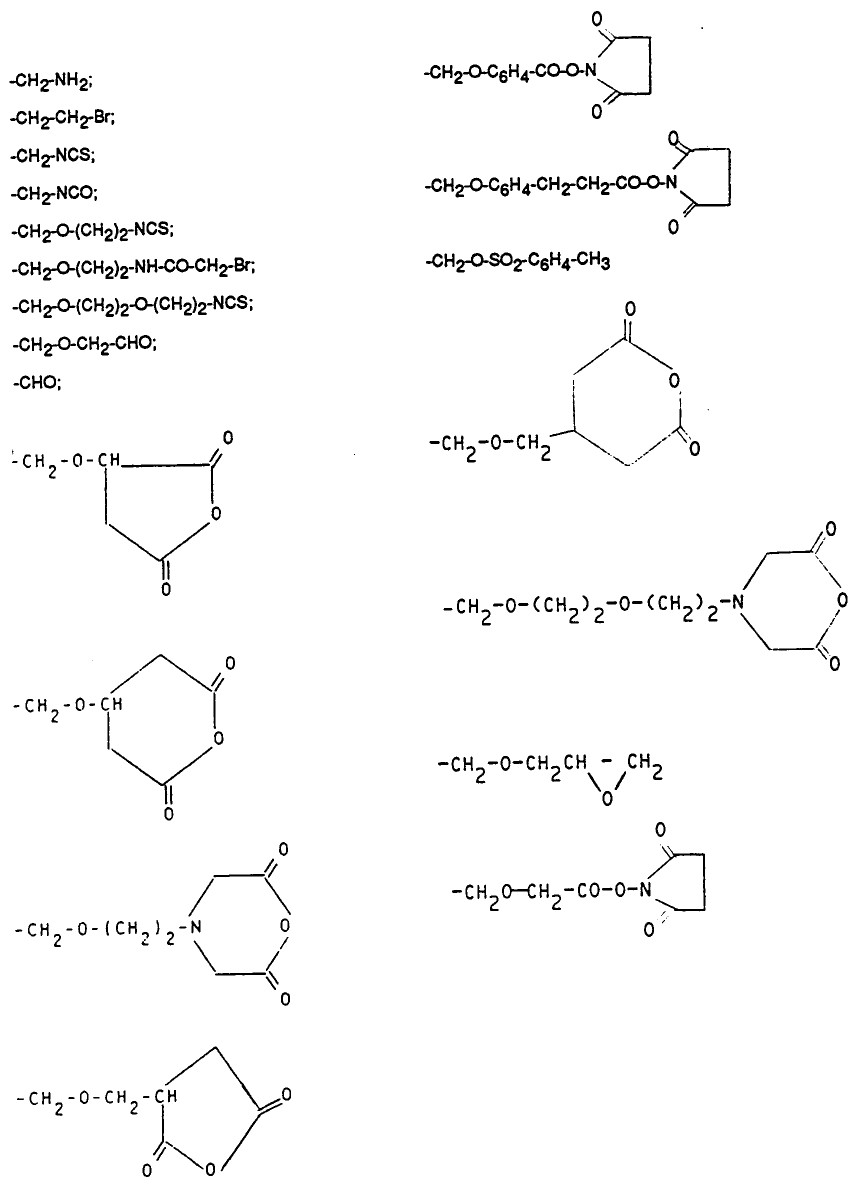

Beispielhaft für die Umsetzung eines Anhydrids sei die Reaktion des Gadolinium-Komplexes des 10-(6-[2,6-Dioxomorpholino]-2-hydroxy-4-oxahexyl)-1,4,7-triscarboxymethyl-1,4,7,10-tetraazacyclododecans mit den jeweils gewünschten, primäre Aminogruppen enthaltenden Backbone-Verbindungen in Wasser oder in Mischungen von Wasser mit z. B. Dioxan, THF, DMF, DMSO oder Acetonitril bei basischem pH, vorzugsweise 8 - 10, d. h. unter Zusatz von Basen wie z. B. Natrium-, Kaliumhydroxid oder Triethylamin, bei Temperaturen von 0-50°C, vorzugsweise bei Raumtemperatur, genannt. Zur vollständigen Umsetzung arbeitet man vorzugsweise mit einem z. B. 2 - 3fachen Überschuß Anhydrid.

Ebenfalls nach literaturbekannten Methoden werden isothiocyanat-, epoxid- und α-halogenacetylderivatisierte Komplexbildner bzw. Komplexe unter pH-Kontrolle in wäßrigem Milieu mit den gewünschten aminhaltigen Backbone-Verbindungen zur Reaktion gebracht.

Als weitere Möglichkeit sei die Umsetzung von endständige Aldehydgruppen aufweisenden Makrocyclen mit den jeweils gewünschten, primäre Aminogruppen enthaltenden Backbone-Verbindungen mit anschließender Reduktion der dabei entstehenden Schiff-Basen analog literaturbekannten Methoden (Synthesis 1975, 135) genannt. Die hierbei generierten sekundären Amine können durch nachfolgende Acylierung oder Alkylierung mit α,β-ungesättigten Estern, Alkylhalogeniden, Anhydriden, Säurehalogeniden oder einer weiteren makrocyclischen Verbindung IA' in tertiäre Amine, Amide oder Thioamide überführt werden. Als beispielhaft genannte, die sekundären Amino-Wasserstoffatome substituierenden Reaktionspartner seien aufgeführt:
Br-CH₂COOH; Cl-CH₂-CH₂-COOH; H₂C=CH-COOCH₃; HOOC-CH=CH-COOCH₃; CH₂=C(COOEt)-CH₂OH; Br-CH₂-SO₃H; Cl-CH₂-CH₂-SO₃H; CH₃-CO-Cl; Cl-CO-CH₂OH; Cl-CO-CHOH-CH₂OH; ClSO₂CH₃; ClSO₂CH₂COOC₂H₅; Br-CO-(CHOH)₄-CH₂OH; Cl-CO-CH₂-COOC₂H₅; 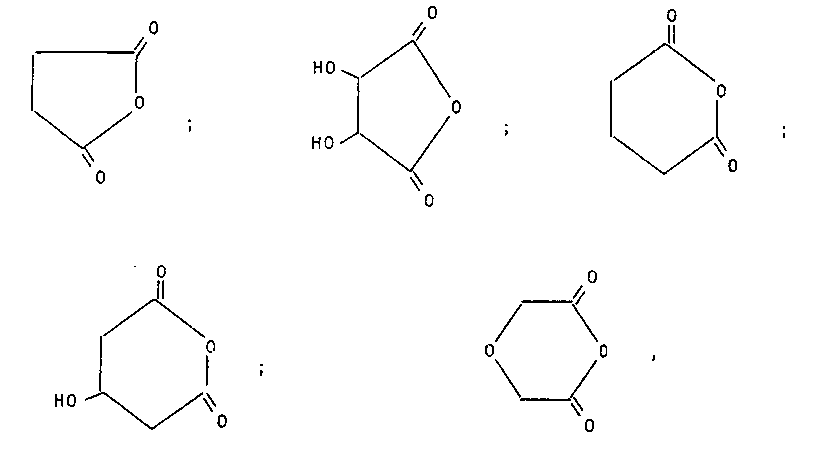 Br-CO-(CH₂)₄-COOC(CH₃)₃; Cl-COCHOH-COOCH₃.

Die dabei als Edukte benötigten Aldehyde können aus den entsprechenden vicinalen Diolen durch Oxidation mit beispielsweise Natriummetaperjodat in wäßriger oder alkoholischer Lösung analog literaturbekannten Methoden (z. B. Makromol Chem. 182, 1641 [1981]) hergestellt werden.

Durch geeignete Reaktionsführung, z. B. Einstellung des pH-Werts oder Zusatz von Aminen, können miteingeführte Estergruppen gewünschtenfalls verseift bzw. aminolysiert werden.
Die Umsetzung von Carboxy-Gruppen enthaltenden Backbone (A')- bzw. makrocyclischen (IA') Verbindungen mit Amino- und Hydroxygruppen enthaltenden makrocyclischen (IA')- bzw. Backbone (A')-Verbindungen erfolgt ebenfalls nach Standardmethoden (Houben-Weyl, Methoden der Organischen Chemie, Georg-Thieme-Verlag, Stuttgart, Bd. XV/2 (1974), 1-364).

Die Reinigung der so erhaltenen makrocyclischen Polymere erfolgt vorzugsweise durch Ultrafiltration mit Membranen geeigneter Porengröße (z. B. Amicon®) oder Gelfiltration an z. B. geeigneten Sephadex®-Gelen.

Die als Edukte benötigten Komplexbildner der allgemeinen Formel IA' können z. B.durch Umsetzung von z. B. aus den Europäischen Patentanmeldungen EP 255 471 und EP 287 465 bekannten tri-CHR¹U' substituierten Tetraazacyclododecan-Derivaten mit Epoxiden der allgemeinen Formel II 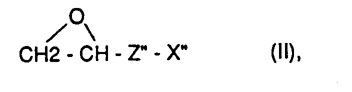 worin
- Z'': für den Rest Z', in dem gegebenenfalls enthaltende Hydroxy-, Carboxy-und Aminogruppen in geschützter Form vorliegen und
- X'': für einen in X' überzuführenden Rest steht,
in Wasser oder in mit Wasser mischbaren Lösungsmitteln wie z. B. Acetonitril, DMF, DMA, Ethanol, Methanol, Dioxan, THF, DMSO, DME oder deren Gemischen bei Temperaturen von 0°C bis 170°C vorzugsweise 20 bis 100°C, innerhalb von 12 bis 48 Stunden, vorzugsweise 12 bis 24 Stunden, unter Zusatz von anorganischen und/oder organischen Basen, wie z. B. Kalium, Natrium,-Lithium-, Calcium-, Barium-, Magnesiumhydroxid, Natrium-, Kaliumcarbonat, Triethyl-, Tripropyl-, Tributylamin-, Pyridin, DMAP, Reillex®, Triton B® und anschließende Umwandlung des Restes X'' in X' hergestellt werden.

Als Hydroxyschutzgruppen kommen alle diejenigen infrage, die sich leicht einführen und später unter Rückbildung der letztlich gewünschten freien Hydroxygruppe auch wieder leicht abspalten lassen. Bevorzugte Schutzgruppen sind Ethergruppen wie zum Beispiel die Benzyl-, 4-Methoxybenzyl-, 4-Nitrobenzyl-, Trityl-, Di- und Triphenylmethyl-, Trimethylsilyl-, Dimethyl-t-butylsilyl-, Diphenyl-t-butylsilylgruppe. Bevorzugt sind die Hydroxygruppen jedoch in Form von Ketalen mit zum Beispiel Aceton, Acetaldehyd, Cyclohexanon oder Benzaldehyd geschützt.

Die Abspaltung der Hydroxyschutzgruppen erfolgt in an sich bekannter Weise, zum Beispiel im Falle eines Benzylethers durch reduktive Spaltung mit Lithium/Ammoniak oder durch hydrogenolytische Spaltung in Gegenwart von zum Beispiel Palladium-Kohle und im Falle einer Ether- oder Ketalspaltung durch Säurebehandlung mit Hilfe von zum Beispiel Kationenaustauschern, Trifluoressigsäure oder Mineralsäuren [siehe z. B. T.W. Greene "Protective Groups in Organic Synthesis", John Wiley and Sons (1981)].

Als Säureschutzgruppen kommen niedere Alkyl-, Aryl- und Aralkylgruppen, beispielsweise die Methyl-, Ethyl-, Propyl-, n-Butyl-, t-Butyl-, Phenyl-, Benzyl-, Diphenylmethyl-, Triphenylmethyl-, bis(p-Nitrophenyl)-methylgruppe, sowie Trialkylsilylgruppen infrage.

Die Abspaltung der Säureschutzgruppen erfolgt nach den dem Fachmann bekannten Verfahren, beispielsweise durch Hydrolyse, Hydrogenolyse, alkalische Verseifung der Ester mit Alkali in wäßrig-alkoholischer Lösung bei Temperaturen von 0 bis 50 °C, saure Verseifung mit Mineralsäuren oder im Fall von z.B. tert.-Butylestern mit Hilfe von Trifluoressigsäure.

Die Aminogruppen sind ebenfalls geschützt, z. B. als Tosylate, Amide, Trifluoracetate, Urethane und Benzylate und werden später ebenfalls nach literaturbekannte Verfahren wieder freigesetzt [vgl. z. B. T. W. Greene "Protective Groups in Organic Synthesis", John Wiley and Sons (1981)].

Die als X' gewünschten funktionellen Gruppen NH₂, NHCOCH₂Hal, NCS und -NCO werden nach der Alkylierung mit einem Epoxin der allgemeinen Formel II, worin X'' für -NO₂ oder -N(CH₂-C₆H₅)₂ steht, nach literaturbekannten Methoden (Europäische Patentanmeldung Publikations-Nr.: 292 689; US-Patent 4,678,667; Bioconjugate Chem. 1990, 1, 59; J. Med. Chem. 1989, 32, 236) generiert.

Die Herstellung der Makrocyclen IA' worin X' für C*O steht (z. B. gemischtes Anhydrid, N-Hydroxysuccinimidester, Acylimidazole, Trimethylsilylester) erfolgt nach literaturbekannten Methoden [Houben-Weyl, Methoden der organischen Chemie, Georg Thieme Verlag, Stuttgart, Band E 5 (1985), 633; Org. React. 12, 157 (1962)] oder wird im experimentellen Teil beschrieben.

Die als Edukte benötigten Epoxide II sind bekannt oder können analog literaturbekannten Methoden hergestellt werden, z. B.durch Umsetzung von Alkoholen mit Epichlorhydrin (Synthesis 1983, 117; Houben-Weyl, Methoden der Organischen Chemie, Georg-Thieme-Verlag Stuttgart, Bd. VI/3 (1965), 369-487) und Epoxidierung von substituierten Allylalkoholen oder ungesättigten Ethern (J. Org. Chem. 35, 215 (1970), Houben-Weyl, Georg-Thieme-Verlag Stuttgart 1965; Tetrahedron Lett. 1965, 849].

Die so erhaltenen Verbindungen der Formel IA' werden nach Reinigung an Kieselgel-Säulen, Reversed-phase-Säulen (RP-18) oder an lonenaustauscher-Säulen (z. B. IRA 67, IR 120, AMB 252) isoliert.

Die verwendeten Backbone-Moleküle A' sind nach literaturbekannten Methoden herzustellen oder auch käuflich zu erwerben. So ist Polyallylamin z. B. bei Aldrich Chemical Company, Polyaminodextrane und Polypeptide wie z. B. Poly-Lysine verschiedener Molmasse, Poly-Asparaginsäure, Poly-Glutaminsäure, Poly-Serin etc. bei Sigma Chemical Co. zu erhalten. Polyaminodextrane sind herstellbar nach US-Patent-Nr. 4,699,784.

Die Herstellung der erfindungsgemäßen Metallkomplexe erfolgt in der Weise, wie sie in der Deutschen Offenlegungsschrift 34 01 052 offenbart worden ist, indem man das Metalloxid oder ein Metallsalz (beispielsweise das Nitrat, Acetat, Carbonat, Chlorid oder Sulfat des Elements der Ordungszahlen 21-29, 31, 32, 37-39, 42-44, 47, 49, 57-83) in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome durch Kationen anorganischer und/oder organischer Basen oder Aminosäuren substituiert.

Die Einführung der gewünschten Metallionen kann dabei sowohl vor als auch nach der Reaktion des Makrocyclus I' mit A' erfolgen. Im Falle der radioaktiv-markierten Polymer-Komplexe wird die Einführung der Radioisotope bevorzugt in der letzten Reaktionsstufe vorgenommen. Falls eine Komplexierung mit unterschiedlichen Ionen gewünscht wird. So können beispielsweise bei der Tumortherapie mit Yttrium-90 zunächst eine für die Kernspintomographie erforderliche Anzahl von Gadoliniumionen eingeführt werden und später am fertigen Konjugat vorhandene Metallatome umkomplexiert oder unbesetzt gebliebene Komplexbildner mit dem Radioisotop markiert werden (siehe Beispiele 13 und 14). Auf diese Weise gelingt es, Diagnostik und Therapie mit Hilfe der erfindungsgemäßen Komplexkonjugate zu kombinieren.

Die Neutralisation eventuell noch vorhandener freier Carboxygruppen erfolgt mit Hilfe anorganischer Basen (z. B. Hydroxiden, Carbonaten oder Bicarbonaten) von z. B. Natrium, Kalium, Lithium, Magnesium oder Calcium und/oder organischer Basen wie unter anderem primärer, sekundärer und tertiärer Amine, wie z. B. Ethanolamin, Morpholin, Glucamin, N-Methyl- und N,N-Dimethylglucamin, sowie basischer Aminosäuren, wie z. B. Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie z. B. niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, diegewünschte Base bereits während der Komplexbildung der Reaktionsmischung zuzusetzen und dadurch einen Verfahrensschritt einzusparen.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusatze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern (wie zum Beispiel Diethylentriaminpentaessigsäure) oder - falls erforderlich - Elektrolyte wie zum Beispiel Natriumchlorid oder - falls erforderlich - Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween^{(R)}, Myrj^{(R)} und/oder Aromastoff(en) zur Geschmackskorrektur (zum Beispiel ätherischen Ölen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 0,1 µMol - 3 Mol/l des Komplexsalzes und werden in der Regel in Mengen von 0,1 µMol - 5 mMol/kg dosiert. Sie sind zur enteralen und parenteralen Applikation bestimmt. Die erfindungsgemäßen Komplexverbindungen kommen zur Anwendung:
1.) für die NMR- und Röntgen-Diagnostik in Form ihrer Komplexe mit den Ionen der Elemente mit den Ordnungszahlen 21 - 29, 42, 44 und 57-83;
2.) für die Radiodiagnostik und Radiotherapie in Form ihrer Komplexe mit den Radioisotopen der Elemente mit den Ordnungszahlen 21, 26, 27, 29, 31, 32, 37-39, 43, 47, 49, 62-64, 67, 70, 71, 75, 77, 79 und 83.

Die erfindungsgemäßen Mittel erfüllen die vielfältigen Voraussetzungen für die Eignung als Kontrastmittel für die Kernspintomographie. So sind sie hervorragend dazu geeignet, nach enteraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100 bis 1000fach besser wasserlöslich sein als für die NMR-Spektroskopie. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,0001 - 5 mMol/kg, vorzugsweise 0,005 - 0,5 mMol/kg, dosiert. Details der Anwendung werden zum Beispiel in H.J. Weinmann et al., Am. J. of Roentgenology 142, 619 (1984) diskutiert.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida, beschrieben.

Eine weitere bildgebende Methode mit Radioisotopen ist die Positronen-Emissions-Tomographie, die positronenemittierende Isotope wie z.B. ⁴³Sc, ⁴⁴Sc, ⁵²Fe, ⁵⁵Co und ⁶⁸Ga verwendet (Heiss, W.D.; Phelps, M.E.; Positron Emission Tomography of Brain, Springer Verlag Berlin, Heidelberg, New York 1983).

Die erfindungsgemäßen Verbindungen können auch in der Radioimmuno- oder Strahlentherapie verwendet werden. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Geeignete β-emittierende Ionen sind zum Beispiel ⁴⁶Sc, ⁴⁷Sc, ⁴⁸Sc, ⁷²Ga, ⁷³Ga und ⁹⁰Y. Geeignete geringe Halbwertzeiten aufweisende α-emittierende Ionen sind zum Beispiel ²¹¹Bi, ²¹²Bi, ²¹³Bi und ²¹⁴Bi, wobei ²¹²Bi bevorzugt ist. Ein geeignetes Photonen- und Elektronenemittierendes Ion ist ¹⁵⁸Gd, das aus ¹⁵⁷Gd durch Neutroneneinfang erhalten werden kann.

Ist das erfindungsgemäße Mittel zur Anwendung in der von R.L. Mills et al. [Nature Vol. 336, (1988), S. 787] vorgeschlagenen Variante der Strahlentherapie bestimmt, so muß sich das Zentralion von einem Mößbauer-Isotop wie beispielsweise ⁵⁷Fe oder ¹⁵¹Eu ableiten.

Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Storung, dem benutzten Metallion und der Art der Methode, z.B. Brachytherapie.

Die erfindungsgemäßen therapeutischen Mittel werden parenteral appliziert.

Details der Anwendung von Radiotherapeutika werden z.B. in R.W. Kozak et al. TIBTEC, Oktober 1986, 262, diskutiert.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Röhrenspannungen für digitale Substraktionstechniken.

Um einen breiten Bereich der Röntgenstrahlung auszunutzen, ist es oft vorteilhaft, zwei oder mehr als zwei verschiedene Metalle im Polymer-Komplex gebunden zu haben.

Im allgemeinen werden die erfindungsgemäßen Mittel für die Anwendung als Röntgenkontrastmittel in Analogie zu zum Beispiel Meglumin-Diatrizoat in Mengen von 0,1 - 5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler - "Einführung in die Röntgendiagnostik", G. Thieme, Stuttgart, New York (1977) diskutiert.

Ein weiterer Vorteil der erfindungsgemäßen makrocyclischen Polymer-Komplexe liegt in der nuklearmedizinischen Anwendung zur Diagnose und/oder Therapie maligner Tumore mittels bispezifischer Antikörper (K. G. Burnett et al., Biotechnology; Appl. Res. 1983, 31, 401). Diese bispezifischen (bifunktionellen) Antikörper sind gegen zwei verschiedene Antigene gerichtet, z. B. gegen ein Tumorantigen und gegen die erfindungsgemäßen Polymerkomplexe. Dadurch kann der Antikörper vor Gabe des radioaktiv markierten Komplexes am bzw. im Tumor lokalisiert werden. Anschließed wird das zur Diagnostik oder zur Therapie bestimmte radioaktiv markierte Polymer verabreicht, welches durch die zweite Spezifität des Antikörpers an der gewünschten Stelle immobilisiert wird. Kürzlich wurde u.a. die gentechnologische Herstellung von humanisierten Maus-IgG's beschrieben (J. L. Phelps et al., J. Immunol. 1990, 145, 1200), die zur Anwendung beim Menschen besonders geeignet sind. Auch bispezifische Antikörper für die Kernresonanz- und Röntgen-Diagnostik können auf diese Weise erhalten werden.

Insgesamt ist es gelungen, neue Komplexbildner, Metallkomplexe und Metallkomplexsalze zu synthetisieren, die neue Möglichkeiten in der diagnostischen und therapeutischen Medizin erschließen. Vor allem die Entwicklung neuartiger bildgebender Verfahren in der medizinischen Diagnostik läßt diese Entwicklung wünschenswert erscheinen.

Die nachfolgenden Beispiele dienen zur näheren Erläuterung des Erfindungsgegenstands.

### Beispiel 1

### a) 1,2-Epoxy-3-dibenzylaminopropan

Zu einer gut gerührten Suspension aus 234,51 g (2,53 mol) Epichlorhydrin und 200 ml 32 % Natronlauge tropft man bei 0°C 100 g (506,9 mmol) Dibenzylamin (gelöst in 300 ml Dichlormethan). Man rührt 2 Stunden bei 0°C, dann 3 Stunden bei Raumtemperatur. Es wird mit 3 l Wasser verdünnt und 3 mal mit 500 ml Dichlormethan extrahiert. Die organischen Phasen werden vereinigt, über Magnesiumsulfat getrocknet und im Vakuum eingedampft. Das zurückbleibende Öl wird an Kieselgel "Flash"-chromatographiert (Laufmittel: Dichlormethan/Hexan/ Aceton: 20/10/3).
Ausbeute: 111,72 g (87 % d. Th.) eines farblosen Öls

| Analyse (bezogen auf wasserfreie Substanz): | | | |
|---|---|---|---|
| C 80,60 | H 7,56 | N 5,53 | ber. |
| C 80,62 | H 7,50 | N 5,48 | |

### b) 10-(3-Dibenzylamino-2-hydroxypropyl)-1,4,7-tris-carboxymethyl-1,4,7,10-tetraazacyclododecan

20 g (78,95 mmol) der Titelverbindung aus Beispiel 1a und 20,51 g (59,21 mmol) 1,4,7-Triscarboxymethyl-1,4,7,10-tetraazacyclododecan (DO3A) werden in einer Mischung aus 50 ml Dioxan und 200 ml Wasser gelöst, und der pH-Wert mit 6N Kalilauge auf pH 10 gebracht. Man rührt 24 Stunden bei 40°C. Man dampft zur Trockene ein, nimmt den Rückstand mit 500 ml Wasser/500 ml Methanol auf und extrahiert 2 mal mit 200 ml tert-Butyl-methylether. Die wässrige Lösung wird mit 5N Salzsäure auf pH3 gestellt und zur Trockene eingedampft. Der Rückstand wird im Vakuum eingeengt und anschließend auf eine Säule aus Poly-(4-vinylpyridin) gegeben. Das Produkt wird mit einer Lösung aus Ethanol/ Wasser 1:1 eluiert. Nach Eindampfen im Vakuum erhält man 22,37 g (63 % d. Th.) eines stark hygroskopischen, glasigen Feststoffes (6,9 % Wasser laut Analyse).

| Analyse (bezogen auf wasserfreie Substanz): | | | |
|---|---|---|---|
| C 62,08 | H 7,56 | N 11,68 | ber. |
| C 62,15 | H 7,61 | N 11,61 | |

### c) Gadolinium Komplex des 10-(3-Dibenzylamino-2-hydroxypropyl)-1,4,7-tris-carboxymethyl-1,4,7,10-tetraazacyclododecans

21 g (35,02 mmol) der Titelverbindung aus Beispiel 1b werden in einer Lösung aus 150 ml entionisiertem Wasser/50 ml Methanol gelöst und 6,35g (17,51 mmol) Gadoliniumoxid zugesetzt. Man kocht 2 Stunden unter Rückfluß. Es werden 3 g Aktivkohle zugesetzt. Die Lösung wird heiß filtriert und das Filtrat im Vakuum zur Trockene eingedampft.
Ausbeute: 25,08 g (95 % d. Th.) eines glasigen Feststoffes (5,2 % Wasser laut Analyse)

| Analyse (bezogen auf wasserfreie Substanz): | | | | |
|---|---|---|---|---|
| C 49,39 | H 5,61 | N 9,29 | Gd 20,86 | ber. |
| C 49,41 | H 5,70 | N 9,25 | Gd 20,88 | |

### d) Gadoliniumkomplex des 10-(3-Amino-2-hydroxypropyl)-1,4,7-triscarboxymethyl-1,4,7,10-tetraazacyclododecans

24 g (31,83 mmol) der Titelverbindung aus Beispiel 1c werden in einer Mischung aus 250 ml entionisiertem Wasser/150 ml Methanol gelöst und 2 g Pearlman's Katalysator(20 % Palladiumhydroxid auf Aktivkohle) zugesetzt. Anschließend wird 24 Stunden bei 50°C hydriert. Man filtriert vom Katalysator ab und dampft das Filtrat im Vakuum ein.
Ausbeute: 17,89 g (98 % d. Th.) eines glasigen Feststoffes (6,4 % Wasser laut Analyse)

| Analyse (bezogen auf wasserfreie Substanz): | | | | |
|---|---|---|---|---|
| C 35,59 | H 5,27 | N 12,21 | Gd 27,41 | ber. |
| C 35,51 | H 5,34 | N 12,16 | Gd 27,36 | |

### e) Gadoliniumkomplex des 10-(3-Isothiocyanato-2-hydroxypropyl)-1,4,7-triscarboxymethyl-1,4,7,10-tetraazacyclododecans

Zu einer Lösung aus 12g (20,92 mmol) der Titelverbindung aus Beispiel 1d in 500 ml entionisiertem Wasser und 20 ml Polyvinylpyridin (Reillex) gibt man eine Lösung aus 4,81 g (41,83 mmol) Thiophosgen in 100 ml Chloroform. Die zweiphasige Lösung wird 10 Minuten bei 40°C, dann eine Stunde bei Raumtemperatur gerührt und filtriert. Die organische Phase wird abgetrennt und die Wasserphase 2 mal mit 200 ml Chloroform nachextrahiert. Anschließend wird die Wasserphase gefriergetrocknet.
Ausbeute: 12,62 g (98 % d. Th.) eines farblosen Pulvers (5,7 % Wasser laut Analyse)

| Analyse (bezogen auf wasserfreie Substanz): | | | | | |
|---|---|---|---|---|---|
| C 35,11 | H 4,58 | N 11,37 | S 5,21 | Gd 25,54 | ber. |
| C 35,04 | H 4,64 | N 11,31 | S 5,15 | Gd 25,48 | |

### f) Thio-ureido-Konjugat aus dem Gd-Komplex des 10-(3-Isothiocyanato-2-hydroxypropyl)-1,4,7-triscarboxymethyl-1,4,7,10-tetraazacyclododecans mit poly-L-Lysin

2,09 g poly-L-Lysin-Hydrobromid [Sigma], 10 mmol, werden in 25 ml H₂O gelöst und zügig über eine Anionenaustauschersäule (10 ml Amberlite® IRA 410, OH⁻-Form) zum Amin freigesetzt. Zu dieser basischen Lösung werden unter Stickstoff 7,39 g (12 mmol) des in Beispiel 1e beschriebenen Isothiocyanat-Gd-Komplexes in fester Form zugegeben und über Nacht bei Raumtemperatur gerührt. Nach Ultrafiltration (Amicon® YM-5-Membran) wird die Leitfähigkeit der Lösung mittels Ionenaustauscher (Amberlite® IR 120, H⁺-Form, und IRA 410, OH⁻-Form) auf ein Minimum eingestellt. Es wird vorn Austauscher abfiltriert und gefriergetrocknet.
Ausbeute: 7,12 g (89 % d. Th.)
H₂O-Gehalt (Karl-Fischer): 7,03 %
Gd-Bestimmung (AAS): 18,72 %
T₁-Relaxivität (H₂O): 11,83 ± 0,42 [l/mmol·sec]
T₁-Relaxivität (Plasma): 11,99 ± 0,57 [l/mmol·sec]

| Analyse (bezogen auf wasserfreie Substanz): | | | | | |
|---|---|---|---|---|---|
| C 38,75 | H 5,42 | Gd 21,14 | N 13,18 | S 4,31 | ber. |
| C 39,11 | H 5,57 | Gd 19,83 | N 12,87 | S 4,01 | |

### Beispiel 2

### a) 1-Dibenzylamino-5,6-epoxy-3-oxahexan

100 g (414 mmol) N-Dibenzylaminoethanol werden in 200 ml Dichlormethan gelöst und bei 0°C zu einer stark gerührten Mischung aus 250 ml 50 % Natronlauge, 7,03 g (20,7 mmol) Tetra-n-butylammoniumhydrogensulfat und 153,4 g (1,657 mol) Epichlorhydrin getropft. Man rührt 8 Stunden bei 0°C über Nacht bei Raumtemperatur. Man verdünnt mit 2 l Wasser und extrahiert 3 mal mit 500 ml Dichlormethan. Die vereinigten organischen Phasen werden über Magnesiumsulfat getrocknet und im Vakuum eingedampft. Das zurückbleibende Öl wird einer Flash-Chromatographie an Kieselgel unterworfen. (Laufmittel: Dichlormethan/Hexan/Aceton 20/10/3).
Ausbeute: 96,12 g (78 % d. Th.) eines farblosen Öls

| Analyse (bezogen auf wasserfreie Substanz): | | | |
|---|---|---|---|
| C 76,74 | H 7,79 | N 4,71 | ber. |
| C 76,68 | H 7,85 | N 4,66 | |

### b) 10-(6-Dibenzylamino-2-hydroxy-4-oxahexyl)-1,4,7-tris-carboxymethyl-1,4,7,10-tetraazacyclododecan

34,34 g (115,47 mmol) der Titelverbindung aus Beispiel 2a und 20 g (57,74 mmol) 1,4,7-Triscarboxymethyl-1,4,7,10-tetraazacyclododecan (DO3A) werden in einer Mischung aus 60 ml Dioxan/350 ml Wasser gelöst und der pH-Wert mit 6N Kalilauge auf pH 10 gestellt. Man rührt 24 Stunden bei 40°C. Es wird wie unter Beispiel 1b beschrieben, aufgearbeitet.
Ausbeute: 26,39 g (71 % d. Th.) eines glasigen Feststoffes (7,1 % Wasser laut Analyse)

| Analyse (bezogen auf wasserfreie Substanz): | | | |
|---|---|---|---|
| C 61,57 | H 7,67 | N 10,88 | ber. |
| C 61,49 | H 7,80 | N 10,79 | |

### c) Gadoliniumkomplex des 10-(6-Dibenzylamino-2-hydroxy-4-oxahexyl)-1,4,7-triscarboxymethyl-1,4,7,10-tetraazacyclododecans

23 g (35,73 mmol) der Titelverbindung aus Beispiel 2b werden in einer Lösung aus 150 ml entionisiertem Wasser/50 ml Methanol gelöst und 6,48 g (17,86 mmol) Gadoliniumoxid zugesetzt. Man kocht 2 Stunden unter Rückfluß. Es werden 3 g Aktivkohle zugesetzt und eine zweite Stunde refluxiert. Die Lösung wird heiß filtriert und das Filtrat im Vakuum zur Trockene eingedampft.
Ausbeute: 27,65 g (97 % d. Th.) eines glasigen Feststoffes (7,8 % Wasser laut Analyse)

| Analyse (bezogen auf wasserfreie Substanz): | | | | |
|---|---|---|---|---|
| C 49,67 | H 5,81 | N 8,78 | Gd 19,71 | ber. |
| C 49,61 | H 5,89 | N 8,71 | Gd 19,61 | |

### d) Gadoliniumkomplex des 10-(6-Amino-2-hydroxy-4-oxahexyl)-1,4,7-triscarboxymethyl-1,4,7,10-tetraazacyclododecans

25 g (31,33 mmol) der Titelverbindung aus Beispiel 2c werden in einer Mischung aus 250 ml entionisiertem Wasser/150 ml Methanol gelöst und 2 g Pearlman's Katalysator (20 % Palladiumhydroxid auf Aktivkohle) zugesetzt. Anschließendwird 24 Stunden bei 50°C hydriert. Man filtriert vom Katalysator ab und dampft das Filtrat im Vakuum ein.
Ausbeute: 19,16 g (99 % d. Th.) eines glasigen Feststoffes (5,7 % laut Analyse)

| Analyse (bezogen auf wasserfreie Substanz): | | | | |
|---|---|---|---|---|
| C 36,94 | H 5,55 | N 11,34 | Gd 25,45 | ber. |
| C 36,88 | H 5,59 | N 11,27 | Gd 25,38 | |

### e) Gadoliniumkomplex des 10-(6-Isothiocyanato-2-hydroxy-4-oxahexyl)-1,4,7-triscarboxymethyl-1,4,7,10-tetraazacyclododecans

Zu einer Lösung aus 15 g (24,28 mmol) der Titelverbindung aus Beispiel 2d in 500 ml entionisiertem Wasser und 20 ml Polyvinylpyridin (Reillex) gibt man eine Lösung aus 5,58 g (48,56 mmol) Thiophosgen in 100 ml Chloroform. Die zweiphasige Lösung wird 10 Minuten bei 40°C, dann eine Stunde bei Raumtemperatur gerührt und filtriert. Die organische Phase wird abgetrennt und die Wasserphase 2 mal mit 200 ml Chloroform extrahiert. Anschließend wird die Wasserphase gefriergetrocknet.
Ausbeute: 15,7 g (98 % d. Th.) eines farblosen Pulvers (6,1 % Wasser laut Analyse)

| Analyse (bezogen auf wasserfreie Substanz): | | | | | |
|---|---|---|---|---|---|
| C 36,41 | H 4,89 | N 10,61 | Gd 23,83 | S 4,86 | ber. |
| C 36,35 | H 4,95 | N 10,51 | Gd 23,71 | S 4,78 | |

### f) Thio-ureido-Konjugat aus dem Gd-Komplex des 10-(6-Isothiocyanato-2-hydroxy-4-oxahexyl)-1,4,7-triscarboxymethyl-1,4,7,10-tetraazacyclododecans mit poly-L-Lysin

2,09 g poly-L-Lysin-Hydrobromid [Sigma], 10 mmol, werden in 25 ml H₂O gelöst und zügig über eine Anionenaustauschersäule (10 ml Amberlite® IRA 410, OH⁻-Form) zum Amin freigesetzt. Zu dieser basischen Lösung werden unter Stickstoff 7,92 g (12 mmol) des in Beispiel 2e beschriebenen Isothiocyanat-Gd-Komplexes gegeben und die Suspension über Nacht bei Raumtemperatur gerührt. Nach Ultrafiltration (Amicon® YM 5-Membran) wird die Leitfähigkeit der Lösung mittels Ionenaustauscher (Amberlite® IR 120, H⁺-Form, und IRA 410, OH⁻-Form) auf ein Minimum eingestellt. Es wird vom Austauscher abfiltriert und gefriergetrocknet.
Ausbeute: 7,49 g (91 % d. Th.)
H₂O-Gehalt (Karl-Fischer): 4,26 %
Gd-Bestimmung (AAS): 18,39 %
T₁-Relaxivität (H₂O): 12,34 ± 0,12 [l/mmol·sec]
T₁-Relaxivität (Plasma): 12,13 ± 0,47 [l/mmol·sec]

| Analyse (bezogen auf wasserfreie Substanz): | | | | | |
|---|---|---|---|---|---|
| C 39,63 | H 5,63 | Gd 19,96 | N 12,44 | S 4,07 | ber. |
| C 40,13 | H 5,84 | Gd 19,07 | N 12,15 | S 3,84 | |

### Beispiel 3

### a) 1-Dibenzylamino-5-hydroxy-3-oxapentan

Eine Mischung aus 50 g (475,56 mmol) 2-(2-Amino-ethoxy)-ethanol und 144,6 g (1,046 mol) Kaliumcarbonat in 600 ml Ethanol/60 ml Wasser wird auf 60°C erhitzt. Zu dieser Mischung tropft man innerhalb 1 Stunde 178,95 g (1,046 mol) Benzylbromid zu und kocht anschließend 2 Stunden unter Rückfluß. Man dampft im Vakuum ein, nimmt den Rückstand mit 1 l Dichlormethan auf und filtriert von den Salzen ab. Das Filtrat wird im Vakuum eingeengt und durch Flash-Chromatographie an Kieselgel gereinigt. (Laufmittel: Dichlormethan/Hexan/Aceton: 10/5/1)
Ausbeute: 127,58 g (94 % d. Th.) eines farblosen Öls

| Analyse (bezogen auf wasserfreie Substanz): | | | |
|---|---|---|---|
| C 75,76 | H 8,12 | N 4,91 | ber. |
| C 75,71 | H 8,18 | N 4,85 | |

### b) 1-Dibenzylamino-8,9-epoxy-3,6-dioxanonan

Zu einer gut gerührten Suspension aus 162,11 g (1,752 mol) Epichlorhydrin, 8,2 g (24,15 mmol) Tetra-n-butylammoniumhydrogensulfat und 250 ml 50 % Natronlauge tropft man bei 0°C eine Lösung aus 125 g (438 mmol) der Titelverbindung aus Beispiel 3a in 200 ml Dichlormethan. Man rührt 8 Stunden bei 0°C, über Nacht bei Raumtemperatur. Es wird mit 2 l Wasser verdünnt und 2 mal mit 500 ml Dichlormethan extrahiert. Die vereinigten organischen Phasen werden über Magnesiumsulfat getrocknet und im Vakuum eingedampft. Das zurückbleibende Öl wird durch Flash-Chromatographie an Kieselgel gereinigt (Laufmittel: Dichlormethan/Hexan/Aceton: 20/10/3).
Ausbeute: 116,5 g (78 % d. Th.) eines farblosen Öls

| Analyse (bezogen auf wasserfreie Substanz): | | | |
|---|---|---|---|
| C 73,87 | H 7,79 | N 4,10 | ber. |
| C 73,78 | H 7,95 | N 4,03 | |

### c) 10-(9-Dibenzylamino-2-hydroxy-4,7-dioxanonyl)-1,4,7-triscarboxymethyl-1,4,7,10-tetraazacyclododecan

39,43 g (115,47 mmol) der Titelverbindung aus Beispiel 3b und 20 g (57,74 mmol) 1,4,7-Triscarboxymethyl-1,4,7,10-tetraazacyclododecan (DO3A) werden in einer Mischung aus 60 ml Dioxan/250 ml Wasser gelöst und der pH-Wert mit 6N Kalilauge auf pH 10 gestellt. Man rührt 24 Stunden bei 40°C. Anschließend wird, wie unter Beispiel 1b beschrieben, aufgearbeitet.
Ausbeute: 28,59 g (72 % d. Th.) eines glasigen Feststoffes (6,3 % Wasser laut Analyse)

| Analyse (bezogen auf wasserfreie Substanz): | | | |
|---|---|---|---|
| C 61,12 | H 7,77 | N 10,18 | ber. |
| C 61,07 | h 7,84 | N 10,05 | |

### d) Gadoliniumkomplex des 10-(9-Dibenzylamino-2-hydroxy-4,7-dioxanonyl)-1,4,7-triscarboxymethyl-1,4,7,10-tetraazacyclododecans

25 g (36,35 mmol) der Titelverbindung aus Beispiel 3c werden in einer Lösung aus 150 ml entionisiertem Wasser/50 ml Methanol gelöst und 6,59 g (18,17 mmol) Gadoliniumoxid zugesetzt. Man kocht 2 Stunden unter Rückfluß. Es werden 3 g Aktivkohle zugesetzt und eine zweite Stunde refluxiert. Die Lösung wird heiß filtriert und das Filtrat im Vakuum zur Trockene eingedampft.
Ausbeute: 30,0 g (98 % d. Th.) eines glasigen Feststoffes (5,4 % Wasser laut Analyse)

| Analyse (bezogen auf wasserfreie Substanz): | | | | |
|---|---|---|---|---|
| C 49,92 | H 5,98 | N 8,32 | Gd 18,67 | ber. |
| C 49,83 | H 5,90 | N 8,34 | Gd 18,58 | |

In analoger Weise erhalt man mit ¹⁵¹Eu₂O₃ den entsprechenden Europium-Komplex.

| Analyse (bezogen auf wasserfreie Substanz): | | | | |
|---|---|---|---|---|
| C 50,24 | H 6,02 | N 8,37 | Eu 18,16 | ber. |
| C 50,17 | H 5,96 | N 8,26 | Eu 18,09 | |

### e) Gadoliniumkomplex des 10-(9-Amino-2-hydroxy-4,7-dioxanonyl)-1,4,7-triscarboxymethyl-1,4,7,10-tetraazacyclododecans

29 g (34,44 mmol) der Titelverbindung aus Beispiel 3d werden in einer Mischung aus 250 ml entionisiertem Wasser/150 ml Methanol und 2 g Pearlman's Katalysator (20 % Palladium-hydroxid auf Aktivkohle) zugesetzt. Anschließend wird 24 Stunden bei 50°C hydriert. Man filtriert vom Katalysator ab und dampft das Filtrat im Vakuum ein.
Ausbeute: 22,56 g (99 % d. Th.) eines glasigen Feststoffes (6,5 % Wasser laut Analyse)

| Analyse (bezogen auf wasserfreie Substanz): | | | | |
|---|---|---|---|---|
| C 38,11 | H 5,79 | N 10,58 | Gd 23,76 | ber. |
| C 38,05 | H 5,86 | N 10,47 | Gd 23,65 | |

### f) Gadoliniumkomplex des 10-(9-Isothiocyanato-2-hydroxy-4,7-dioxanonyl)-1,4,7-triscarboxymethyl-1,4,7,10-tetraazacyclododecans

Zu einer Lösung aus 15 g (22,66 mmol) der Titelverbindung aus Beispiel 3e in 500 ml entionisiertem Wasser und 20 ml Polyvinylpyridin (Reillex) gibt man eine Lösung aus 5,21 g (45,33 mmol) Thiophosgen in 100 ml Chloroform. Die zweiphasige Lösung wird 10 Minuten bei 40°C, dann eine Stunde bei Raumtemperatur gerührt und filtriert. Die organische Phase wird abgetrennt und die Wasserphase 2 mal mit 200 ml Chloroform extrahiert. Anschließend wird die Wasserphase gefriergetrocknet.
Ausbeute: 15,64 g (98 % d. Th.) eines farblosen Pulvers (5,9 % Wasser laut Analyse)

| Analyse (bezogen auf wasserfreie Substanz): | | | | | |
|---|---|---|---|---|---|
| C 37,54 | H 5,15 | N 9,95 | Gd 22,34 | S 4,55 | ber. |
| C 37,49 | H 5,11 | N 9,91 | Gd 22,27 | S 4,61 | |

### g) Thio-ureido-Konjugat aus dem Gd-Komplex des 10-(9-Isothiocyanato-2-hydroxy-4,7-dioxanonyl)-1,4,7-triscarboxymethyl-1,4,7,10-tetraazacyclododecans mit poly-L-Lysin

2,09 g poly-L-Lysin-Hydrobromid [Sigma], 10 mmol, werden in 25 ml H₂O gelöst und zügig über eine Anionenaustauschersäule (10 ml Amberlite® IRA 410, OH⁻-Form) zum Amin freigesetzt. Zu dieser Lösung werden unter Stickstoff 8,45 g (12 mmol) des in Beispiel 3f beschriebenen Isothiocyanat-Gd-Komplexes in fester Form zugegeben und über Nacht bei Raumtemperatur gerührt. Nach Ultrafiltration (Amicon® YM-5-Membran) wird die Leitfähigkeit der Lösung mittels Ionenaustauscher (Amberlite® IR 120, H⁺-Form, und IRA 410, OH⁻-Form) auf ein Minimum eingestellt. Es wird vom Austauscher abfiltriert und gefriergetrocknet.
Ausbeute: 7,24 g (81,4 % d. Th.)
H₂O-Gehalt (Karl-Fischer): 6,50 %
Gd-Bestimmung (AAS): 15,89 %
T1-Relaxivität (H₂O): 11,75 ± 0,37 [l/mmol·sec]
T1-Relaxivität (Plasma): 13,07 ± 0,14 [l/mmol·sec]

| Analyse (bezogen auf wasserfreie Substanz): | | | | | |
|---|---|---|---|---|---|
| C 40,42 | H 5,81 | Gd 18,90 | N 11,78 | S 3,85 | ber. |
| C 40,33 | H 5,99 | Gd 17,23 | N 11,95 | S 3,47 | |

### Beispiel 4

### a) 10-[2,6,7-Trihydroxy-4-oxa-heptyl]-1,4,7-tris-carboxymethyl-1,4,7,10-tetraazacyclododecan

19,56g (103,92 mmol) 2,2-Dimethyl-4-(2',3'-epoxy)-propoxy-methyl-1,3-dioxolan und 10g (26,86 mmol) 1,4,7-Triscarboxymethyl-1,4,7,10-tetraazacyclododecan (DO3A) werden in einer Mischung aus 50 ml Dioxan/80 ml Wasser gelöst, und der pH-Wert mit 6N Kalilauge auf pH 10 gebracht. Man rührt 24 Stunden bei 70°C. Man dampft zur Trockene ein, nimmt den Rückstand mit 200 ml Wasser/50 ml Methanol auf und extrahiert 2 mal mit 100 ml tert.-Butyl-methylether. Die wäßrige Lösung wird mit 5N-Salzsäure auf pH 3 gestellt und zur Trockene eingedampft. Der Rückstand wird mit 200 ml Methanol/80 ml Dichlormethan ausgekocht (extrahiert). Man kühlt im Eisbad ab und filtriert vom ausgefallenen Kaliumchlorid ab. Das Filtrat wird im Vakuum eingedampft, der Rückstand in 45 ml Wasser/20 ml Ethanol gelöst und anschließend auf eine Säule aus Poly-(4-vinylpyridin) gegeben. Das Produkt wird mit einer Lösung aus Ethanol/Wasser 1:3 eluiert. Nach Eindampfen im Vakuum wird der Rückstand an einer Reversed Phase-Säule (RP 18/Laufmittel= Gradient aus Wasser/Tetrahydrofuran) chromatographiert. Nach Eindampfen der Hauptfraktion erhält man 10,13 g (71 % d. Th.) eines stark hygroskopischen, glasigen Feststoffes.

| Analyse (bezogen auf wasserfreie Substanz): | | | |
|---|---|---|---|
| C 48,57 | H 7,74 | N 11,33 | ber. |
| C 48,46 | H 7,81 | N 11,24 | |

### b) Gd-Komplex des 10-(2,6,7-Trihydroxy-4-oxa-heptyl)-1,4,7-tris-carboxymethyl-1,4,7,10-tetraazacyclododecans

8,56g (17,3 mmol) der Titelverbindung aus Beispiel 4a werden in 50 ml entionisiertem Wasser gelöst und 3,13g (8,65 mmol) Gadoliniumoxid zugesetzt. Es wird 3 Stunden auf 90°C erhitzt. Die abgekühlte Lösung wird eine Stunde mit 3 ml saurem Ionenaustauscher (AMB 252c) und 3 ml schwach basischem Austauscher (IRA 67) gerührt. Es wird vom Austauscher abfiltriert und das Filtrat gefriergetrocknet.
Ausbeute: 11,0 g (98 % d. Th.) eines farblosen amorphen Pulvers

| Analyse (bezogen auf wasserfreie Substanz): | | | | |
|---|---|---|---|---|
| C 37,03 | H 5,44 | N 8,64 | Gd 24,24 | ber. |
| C 37,00 | H 5,51 | N 8,57 | Gd 24,18 | |

### c) Gd-Komplex des N⁵-{6-[4,7,10-Tris-(carboxymethyl)-1,4,7,10-tetraazacyclododecan-1-yl]-5-hydroxy-3-oxa-hexyl}-poly-L-Lysins

38,93 g (60 mmol) des Gd-Komplexes aus Beispiel 4b werden in 400 ml Methanol gelöst, mit 12,83 g (60 mmol) NalO₄ versetzt und 2 Stunden unter Lichtausschluß gerührt. Dann wird vom Ungelösten abfiltriert und das Filtrat gefriergetrocknet. Das Lyophilisat wird in möglichst wenig Wasser gelöst und mit Ethanol/Ether (3:1) umgefällt. Der Niederschlag wird abgesaugt, mit 4,94 g (30 mmol) poly-L-Lysin-Hydrochlorid in 750 ml Puffer pH 9,0 (Riedel de Haën, Borax/HCl) gelöst und nach Zugabe von 7,55 g (120 mmol) Natriumcyanoborhydrid 6 Tage bei Raumtemperatur unter Lichtausschluß gerührt. Die Lösung wird anschließend ultrafiltriert (Amicon YM-5-Membran) und dann gefriergetrocknet.
Ausbeute: 15,0 g (61,0 % d. Th.)
H2O-Gehalt (Karl-Fischer): 11,1 %
Gd-Bestimmung (AAS): 15,76 %
T₁-Relaxivität (H₂O): 18,30 ± 0,14 [l/mmol·sec]
T₁-Relaxivität (Plasma): 18,20 ± 0,33 [l/mmol·sec]

### Beispiel 5

### Gd-Komplex des N⁵-{6-[4,7,10-Tris-(carboxymethyl)-1,4,7,10-tetraazacyclododecan-1-yl]-5-hydroxy-3-oxa-hexyl}-N⁵-(2-Carboxyethyl)-poly-L-Lysins

2,20 g des in Beispiel 4c beschriebenen Polymers mit sekundärem Amin in den Verknüpfungen zwischen Komplex und poly-Lysin werden in 25 ml Methanol/H₂O (5:1) suspendiert und eine Mischung aus 20 ml (220 mmol) Methylacrylat und 20 ml Methanol zugegeben und 3 Tage bei Raumtemperatur gerührt. Die Lösung wird im Vakuum eingedampft, der Rückstand in 20 ml 1 N NaOH gelöst und 3 Stunden bei Raumtemperatur verseift. Anschließend wird mit verdünnter HCl neutralisiert und die Lösung über eine Amicon-Ultrafiltrationsmembran YM5 entsatzt und schließlich gefriergetrocknet.
Ausbeute: 2,20 g
H₂O-Gehalt (Karl-Fischer): 5,76 %
Gd-Bestimmung (AAS): 15,42 %
T1-Relaxivität (H₂O): 12,39 ± 0,37 [l/mmol·sec]
T1-Relaxivität (Plasma): 12,87 ± 0,09 [l/mmol·sec]
Die Papier-Elektrophorese des Polymers bei pH 9,0 (0,05 M Borax) und 10 V/cm zeigt eine Wanderung zur Anode, während die Ausgangsverbindung (Beispiel 4c) unter den gleichen Bedingungen zur Kathode wandert.

### Beispiel 6

### Gd-Komplex des N⁵-{6-[4,7,10-Tris-(carboxymethyl)-1,4,7,10-tetraazacyclododecan-1-yl]-5-hydroxy-3-oxa-hexyl}-N⁵-(2-Dicarboxyethyl)-poly-L-Lysins

Zu 14,3 g (110 mmol) Maleinsäure-monomethylester (Tokyo Chemical Co. Ltd.) in 15 ml Methanol werden unter Eiskühlung 23 ml Triethylamin zugetropft. Man läßt auf Raumtemperatur erwärmen und tropft diese Lösung zu 2,2 g des in Beispiel 4c beschriebenen Polymers, die in 25 ml Methanol/H₂O (5:1) suspendiert vorgelegt werden und läßt 3 Tage bei Raumtemperatur rühren. Dann wird im Vakuum eingedampft, mit Diethylether verrührt, vom abgeschiedenen Öl abdekantiert, der verbleibende Rückstand in 20 ml 1N NaOH gelöst und 3 Stunden bei Raumtemperatur verseift. Anschließend wird mit verdünnter HCl neutralisiert und die Lösung über eine Amicon-Ultrafiltrationsmembran YM5 entsalzt und schließlich gefriergetrocknet.
Ausbeute: 2,18 g
H₂O-Gehalt (Karl-Fischer): 6,3 %
Gd-Bestimmung (AAS): 15,97 %
T₁-Relaxivität (H₂O): 11,77 ± 0,29 [l/mmol·sec]
   (Plasma): 12,07 ± 0,15 [l/mmol·sec]

### Beispiel 7

### Gd-Komplex des N⁵-{6-[4,7,10-Tris-(carboxymethyl)-1,4,7,10-tetraazacyclododecan-1-yl]-5-hydroxy-3-oxa-hexyl}-N⁵-(2-Carboxymethyl)-poly-L-Lysins

2,20 g des in Beispiel 4c beschriebenen Polymers werden in 25 ml H₂O gelöst und durch Zugabe von 1N NaOH auf pH 10,0 eingestellt. Dazu wird bei 50°C langsam eine Lösung von 2,6 g (22 mmol) Natrium-chloracetat in 20 ml H₂O zugetropft und durch Zugabe von NaOH der pH-Wert bei 10 gehalten. Nach beendeter Zugabe wird über Nacht bei dieser Temperatur gerührt, anschließend mit verdünnter Salzsäure neutralisiert und die Lösung über eine Amicon-Ultrafiltrationsmembran YM5 entsalzt. Nach Gefriertrocknung erhält man 2,27 g flockiges Pulver.
H₂O-Gehalt (Karl-Fischer): 8,3 %
Gd-Bestimmung (AAS): 16,40 %
T1-Relaxivität (H₂O): 12,13 ± 0,19 [l/mmol·sec]
T1-Relaxivität (Plasma): 13,29 ± 0,34 [l/mmol·sec]

### Beispiel 8

### Gd-Komplex des N⁵-{6-[4,7,10-Tris(carboxymethyl)-1,4,7,10-tetraazacyclododecan-1-yl]-5-hydroxy-3-oxahexyl}-N⁵-(Carboxymethoxyacetyl)-poly-L-Lysins

2,20 g des in Beispiel 4c beschriebenen Polymers werden in 25 ml H₂O gelöst und durch Zugabe von 1 N NaOH auf pH 8,5 eingestellt. Dazu werden 1,04 g (9 mmol) Diglykolsäure-anhydrid (Fluka) portionsweise in fester Form zugegeben, wobei der pH-Wert gleichzeitig durch Zugabe von 1 H NaOH zwischen und 8 und 9 gehalten wird. Nach beendeter Zugabe wird 15 Minuten nachgerührt, mit verdünnter Salzsäure neutralisiert, ultrafiltriert (Amicon YM5) und schließlich gefriergetrocknet.
Ausbeute: 2,37 g
H₂O-Gehalt (Karl-Fischer): 6,7 %
Gd-Bestimmung (AAS): 14,93 %
T1-Relaxivitat (H₂O): 13,05 ± 0,37 [l/mmol·sec]
T1-Relaxivität (Plasma): 12,95 ± 0,18 [l/mmol·sec]

### Beispiel 9

### Thio-ureido-Konjugat aus dem Gd-Komplex des N⁵-{6-[4,7,10-Tris(carboxymethyl)-1,4,7,10-tetraazacyclododecan-1-yl]-5-hydroxy-3-oxa-hexyl}-poly-L-Lysins mit dem Gd-Komplex des 10-(6-Isothiocyanato-2-hydroxy-4-oxahexyl)-1,4,7-tris(carboxymethyl)-1,4,7,10-tetraazacyclododecans

2,20 g des in Beispiel 4c beschriebenen Polymers werden in 25 ml H₂O gelöst. Dazu werden unter Stickstoff portionsweise in fester Form 3,09 g (4,7 mmol) des in Beispiel 2e beschriebenen Isothiocyanat-Gd-Komplexes gegeben und über Nacht bei Raumtemperatur gerührt. Nach Ultrafiltration (Amicon YM5) wird die Leitfähigkeit der Lösung mittels Ionenaustauscher (Amberlite IR 120, H⁺-Form, IRA 410, OH⁻-Form) auf ein Minimum eingestellt. Es wird vom Austauscher abfiltriert und gefriergetrocknet.
Ausbeute: 3,43 g
H₂O-Gehalt (Karl-Fischer): 8,1 %
Gd-Bestimmung (AAS): 18,47 %
T1-Relaxivität (H₂O): 13,79 ± 0,25 [l/mmol·sec]
T1-Relaxivität (Plasma): 14,51 ± 0,13 [l/mmol·sec]

### Beispiel 10

### a) 10-(2,3,4-Trihydroxybutyl)-1,4,7-triscarboxymethyl-1,4,7,10-tetraazacyclododecan

10,0 g (28,87 mmol) 1,4,7-Triscarboxymethyl-1,4,7-10-tetraazacyclododecan (DO3A) werden in 40 ml Wasser gelöst und der pH mit 5 N Natronlauge auf 13 eingestellt. Man fügt eine Lösung aus 6,24 g (43,30 mmol) 2-(2,2-Dimethyl-1,3-dioxolan-4-yl)-ethylenoxid (DE 3 150 917) in 10 ml Dioxan zu und rührt 24 Stunden bei Raumtemperatur. Es wird mit 60 ml Wasser verdünnt und dreimal mit 50 ml Ether extrahiert. Die wäßrige Phase wird mit 10 %iger Salzsäure auf pH 2 gebracht und eingedampft. Der Rückstand wird in etwas Wasser gelöst und auf eine Kationenaustauschersäule (IR 120) gegeben. Nach Spülung mit Wasser wird der Ligand mit 0,5 normaler wäßriger Ammoniak-Lösung eluiert. Die Fraktionen werden eingedampft, das Ammoniumsalz mit wenig Wasser aufgenommen und über eine Anionenaustauschersäule (IRA 67) gegeben. Man wäscht zuerst mit Wasser und elulert dann mit 0,5 normaler wäßriger Ameisensäure. Man dampft im Vakuum ein, löst den Rückstand in wenig heißem Methanol und gibt Aceton hinzu, wobei die Titelverbindung auskristallisiert.
Ausbeute: 11,31 g (87 % d. Th.) eines weißen hygroskopischen Pulvers
H₂O-Gehalt (Karl-Fischer): 11,1 %

| Analyse (bezogen auf wasserfreie Substanz): | | | |
|---|---|---|---|
| C 47,99 | H 7,61 | N 12,44 | ber. |
| C 47,93 | H 7,67 | N 12,40 | |

### b) Gadolinium-Komplex des 10-(2,3,4-Trihydroxybutyl)-1,4,7-triscarboxymethyl-1,4,7,10-tetraazacyclododecans

10,0 g (22,2 mmol) der nach Beispiel 10a erhaltenen Verbindung werden in 60 ml entionisiertem Wasser gelöst und 4,02 g (11,1 mmol) Gadoliniumoxid zugesetzt. Man erwärmt 3 Stunden auf 90°C. Die abgekühlte Lösung wird mit je 2 ml saurem Ionenaustauscher (IR 120) und 2 ml basischem Austauscher(IRA 410) 1 Stunde bei Raumtemperatur gerührt. Man filtriert vom Austauscher ab und kocht das Filtrat mit Aktivkohle kurz auf. Nach Filtrieren und Gefriertrocknung erhält man ein weißes, amorphes Pulver.
Ausbeute: 12,76g (95 % d. Th.)
H₂O-Gehalt (Karl-Fischer): 12,3 %

| Analyse (bezogen auf wasserfreie Substanz): | | | | |
|---|---|---|---|---|
| C 35,73 | H 5,17 | Gd 25,99 | N 9,26 | ber. |
| C 35,68 | H 5,24 | Gd 25,93 | N 9,21 | |

### c) Gd-Komplex des N⁵-{3-[4,7,10-Tris(carboxymethyl)-1,4,7,10-tetraazacyclododecan-1-yl]-2-hydroxypropyl)-poly-L-Lysins

13,8 g (20 mmol) des Gd-Komplexes aus Beispiel 10b werden in 120 ml Methanol gelöst, mit 4,28 g (20 mmol) NalO₄ versetzt und 4 Stunden unter Lichtanschluß verrührt. Dann wird vom Ungelösten abfiltriert und das Filtrat gefriergetrocknet. Das Lyophilisat wird in möglichst wenig Wasser gelöst und mit Ethanol/Ether (3:1) umgefällt. Der Niederschlag wird abgesaugt, mit 2,47 g (15 mmol) poly-L-Lysin-Hydrochlorid in 350 ml Puffer pH 9,0 (Riedel de Haën, Borax/HCl) gelöst und nach Zugabe von 3,78 g (60 mmol) Natriumcyanoborhydrid 6 Tage bei Raumtemperatur unter Lichtanschluß gerührt. Die Lösung wird anschließend ultrafiltriert (AMICON YM5-Membran) und dann gefriergetrocknet.
Ausbeute: 7,29 g (71 % d. Th.)
H₂O-Gehalt (Karl-Fischer): 3,1 %
Gd-Bestimmung (AAS): 15,98 %
T1-Relaxivität (H₂O): 12,33 ± 0,19 [l/mmol·sec]
T1-Relaxivität (Plasma): 12,75 ± 0,08 [l/mmol·sec]

### Beispiel 11

### Gd-Komplex des N⁵-{3-[4,7,10-Tris(carboxymethyl)-1,4,7,10-tetraazacyclododecan-1-yl]-2-hydroxypropyl}-N⁵-(Carboxymethoxyacetyl)-poly-L-ysins

1,7 g des in Beispiel 10c beschriebenen Polymers werden in 20 ml H₂O gelöst und durch Zugabe von 1 N NaOH auf pH 8,5 eingestellt. Dazu werden 772 mg (6 mmol) Diglykolsäure-anhydrid (Fluka) portionsweise unter Rühren zugegeben, wobei der pH-Wert durch Zugabe von 1 N NaOH zwischen pH 8,0 und 9,0 gehalten wird. Nach beendeter Zugabe wird 15 Minuten nachgeführt, mit verdünnter Salzsäure neutralisiert, ultrafiltriert (AMICON YM5) und schließlich gefriergetrocknet.
Ausbeute: 1,90 g
H₂O-Gehalt (Karl-Fischer): 7,3 %
Gd-Bestimmung (AAS): 14,79 %
T1-Relaxivität (H₂O): 12,39 ± 0,35 [l/mmol·sec]
T1-Relaxivität (Plasma): 13,18 ± 0,21 [l/mmol·sec]

### Beispiel 12

### a) 2,3-Epoxy-1-[4-(2-ethoxycarbonylethyl)-phenoxy]-propan

Zu 24,03 g (123,74 mmol) 2-(4-Hydroxyphenyl)-propansäureethylester in 400 ml Dimethylformamid gibt man 3,56 g (148,5 mmol) Natriumhydrid zu und rührt 1 Stunde bei Raumtemperatur (unter Stickstoff). Man gibt 34,35 g (371,22 mmol) Epichlorhydrin zu und erwärmt anschließend 24 Stunden bei 70°C. Es wird im Eisbad auf 0°C abgekühlt und vorsichtig 800 ml Wasser zugegeben. Anschließend wird 2 mal mit je 350 ml Ether extrahiert. Die vereinigten Etherphasen werden 1 mal mit 300 ml Wasser gewaschen, über Magnesiumsulfat getrocknet und im Vakuum eingedampft. Der Rückstand wird an Kieselgel chromatographiert (Laufmittel: Hexan/Essigester=20:1)
Ausbeute: 20,75 g (67 % d. Th.) eines farblosen Öls

| Analyse (bezogen auf wasserfreie Substanz): | | |
|---|---|---|
| C 67,18 | H 7,25 | ber. |
| C 67,09 | H 7,32 | |

### b) 10-[2-Hydroxy-3-(4-(2-carboxyethyl)-phenoxy)-propyl]-1,4,7-tris(carboxymethyl)-1,4,7,10-tetraazacyclododecan

10 g (28,87 mmol) 1,4,7-Triscarboxymethyl-1,4,7,10-tetraazacyclododecan (= DO3A) und 12,99 g (51,96 mmol) 2,3-Epoxy-1-[4-(2-ethoxycarbonylethyl)-phenoxy]-propan werden in 80 ml Dioxan/60 ml Wasser gegeben und mit 6N Kalilauge auf pH 14 gestellt. Man rührt 12 Stunden bei Raumtemperatur. Anschließend wird 2 Stunden unter Rückfluß erhitzt. Man stellt mit 5N Salzsäure auf pH 7 und dampft im Vakuum zur Trockene ein. Der Rückstand wird in 200 ml Ethanol/50 ml Chloroform bei 60°C ausgerührt. Das ausgefallene Kaliumchlorid wird abfiltriert und das Filtrat im Vakuum eingedampft. Der Rückstand wird an einer Reversed-Phase-Säule aufgereinigt (RP-18, Waschen mit Wasser, dann Eluieren mit Tetrahydrofuran/Wasser= 2:1). Die Hauptfraktionen werden im Vakuum eingedampft.
Ausbeute: 7,88 g (48 % d.Th.) eines glasigen Feststoffes

| Analyse (bezogen auf wasserfreie Substanz): | | | |
|---|---|---|---|
| C 54,92 | H 7,09 | N 9,85 | ber. |
| C 54,87 | H 7,15 | N 9,79 | |

### c) Gadolinium-Komplex des 10-[2-Hydroxy-3-(4-(2-carboxyethyl)-phenoxy)-propyl]-1,4,7-tris(carboxymethyl)-1,4,7,10-tetraazacyclododecans

7,5 g (13,19 mmol) der Titelverbindung aus Beispiel 12b werden in 50 ml entionisiertem Wasser gelöst und 2,39 g (6,59 mmol) Gadoliniumoxid zugesetzt. Es wird 3 Stunden auf 90°C erhitzt. Die abgekühlte Lösung wird eine Stunde mit 7 ml schwach saurem Ionenaustauscher (AMB 252c) bei Raumtemperatur gerührt. Es wird abfiltriert und gefriergetrocknet.
Ausbeute: 8,96 % (94 % d. Th.) eines farblosen, amorphen Pulvers

| Analyse (bezogen auf wasserfreie Substanz): | | | | |
|---|---|---|---|---|
| C 43,20 | H 5,16 | N 7,75 | Gd 21,75 | ber. |
| C 43,13 | H 5,29 | N 7,86 | Gd 21,53 | |

### d) Gd-Komplex des N⁵-{3-[4-(3-(4,7,10-Tris-carboxymethyl-1,4,7,10-tetraaza-cyclododecan-1-yl)-2-hydroxypropoxy)-phenyl]-propionyl}-poly-L-Lysins

10,84 g (15 mmol) der in Beispiel 12c beschriebenen Komplexsäure werden in 50 ml DMF/H₂O (1:1) gelöst, mit 6,68 g (15 mmol) N-Cyclohexyl-N'-(2-morpholinoethyl)-carbodiimid Metho-p-toluolsulfonat [Aldrich] versetzt und zu einer Lösung von 2,09 g (10 mmol) analog Beispiel 1f zum Amin freigesetztem poly-L-Lysin-Hydrobromid gegeben. Die Reaktionsmischung wird 24 Stunden bei - Raumtemperatur gerührt, über eine Ultrafiltration (AMICON YM5-Membran) von niedermolekularen Substanzen befreit und das Retentat gefriergetrocknet.
Ausbeute: 5,98 g (74 % d. Th.)
H₂O-Gehalt (Karl-Fischer): 6,39 %
Gd-Bestimmung (AAS): 17,13 %
T1-Relaxivität (H₂O): 12,73 ± 0,45 [l/mmol·sec]
T1-Relaxivität (Plasma): 14,51 ± 0,22 [l/mmol·sec]

### Beispiel 13

### Indium-(111)-markierter Gd-Komplex des N⁵-{6-[4,7,10-Tris-(carboxymethyl)-1,4,7,10-tetraaracyclododecan-1-yl]-5-hydroxy-3-oxahexyl}-N⁵-(Carboxymethoxyacetyl)-poly-L-Lysins

1 mg der Titelverbindung aus Beispiel 8 in 150 µl Wasser (pH 4,0/Salzsäure) werden mit 30 µl einer Indium(111)chlorid-Lösung (50 mCi/ml in 0,05 M Salzsäure) versetzt und 3 Stunden bei 50°C gehalten. Anschließend wird mit 0,01 N Natronlauge auf pH 6 gebracht und 6 µl einer 0,01 M Di-Natrium-EDTA-Lösung zugesetzt (um freies In und Gd zu binden). Der so markierte Polymer-Komplex wird mittels Ausschlußchromatographie (HPLC/TSK-400/Puffer MES·Cl, pH 6,2) aufgereinigt und kann für radiodiagnostische Untersuchungen verwendet werden.

### Beispiel 14

### Yttrium(90)-markierter Gd-Komplex des N⁵-{6-[4,7,10-Tris-(carboxymethyl)-1,4,7,10-tetraazacyclododecan-1-yl]-5-hydroxy-3-oxahexyl}-N⁵-(Carboxymethoxyacetyl)-poly-L-Lysins

In analoger Weise (siehe Beispiel 13) kann ein zur Radiotherapie geeigneter Y-(90)-Polymer-Komplex der Titelverbindung aus Beispiel 8 hergestellt werden, wenn Yttrium(90)chlorid anstelle In(111)chlorid verwendet wird.

## Patentansprüche

1. Polymer-Verbindungen der allgemeinen Formel I
(M)ₙA (I),
worin
M für den Rest eines makrocyclischen Komplexbildners,
A für ein Backbone-Molekül, das ein Defizit von n Amino-, n Hydroxy- oder n Carboxygruppen aufweist,
n für die Zahlen 1 bis 400,
M unabhängig voneinander für Komplexbildner der allgemeinen Formel IA steht 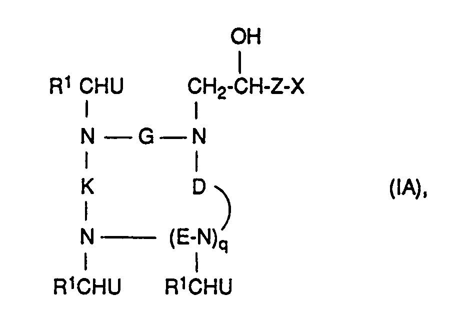 wobei
D, E, G, K, die gleich oder verschieden sein können, jeweils für die Gruppe -(CH₂)ₒ- mit o in der Bedeutung der Ziffern 2,3,4 oder 5
q für die Ziffern 0, 1 oder 2,
R¹ für ein Wasserstoffatom, eine verzweigte oder unverzweigte C₁-C₄-Alkyl-, Phenyl-, C₇-C₁₂-Aralkylgruppe,
U für eine CO₂H- oder PO₃H₂-Gruppe,
Z für eine gegebenenfalls Imino-, Phenylen-, Phenylenoxy-, Phenylenimino-, Amid-, Hydrazid-, Carbonyl-, Estergruppe(n), Sauerstoff-, Schwefel- und/oder Stickstoff-Atom(e) enthaltende, gegebenenfalls durch Hydroxy-, Mercapto-, Oxo-, Thioxo-, Carboxy-, Ester- und/oder Aminogruppe(n) substituierte geradkettige, verzweigte, gesättigte oder ungesättigte C₁-C₂₀-Alkylengruppe,
X für eine -CONH-, -NHCO-, -NHCSNH-, -NHCONH-, -CO₂-, -O-, -OCO-, -COCH₂O-, -NR²CH₂CONH-, -NHCS-, -NR²CH₂COO-, -OCHR²CONH-,-OCHR²COO-, wobei R² für ein Wasserstoffatom oder die Gruppe -(CH₂)pCOOH mit p in der Bedeutung der Ziffern 1-5 steht,und für eine - NR-gruppe, worin R ein Wasserstoffatom, einen gegebenenfalls durch 1 bis 3 Carboxy-, 1 bis 3 Sulfonsäuren, 1 bis 5 Hydroxy-, 1 bis 5 C₁-C₁₀-Alkoxy-, 1 bis 5 C₆-C₁₀-Aryloxy-, 1 bis 5 C₇-C₁₁-Aralkoxy-, 1 bis 5 Ester-, 1 bis 5 Amidgruppen substituierten und/oder gegebenenfalls 1 bis 3 Carbonyl-, 1 bis 3 Ester-, 1 bis 3 Amid-, eine Sulfonylgruppe, 1 bis 10 Sauerstoff-, 1 bis 4 Stickstoffatome enthaltenden C₁-C₂₀-Kohlenwasserstoffrest oder ein zweites Molekül IA, das mit dem ersten nicht identisch sein muß, bedeutet, stehen, deren vollständig oder unvollständig metallierte Komplexe mit den Elementen der Ordnungszahlen 21-29, 31, 32, 37-39, 42-44 ,49, 57-83 sowie deren Salze.

2. Verbindungen gemäß Anspruch 1, dadurch gekennzeichnet, daß A für Polypeptide, Polyallylamine, Poly [N(-2-aminoethyl)] methacrylamide und Polyaminkohlenhydrate, die jeweils ein Defizit von n Amino-, n Carboxy- oder n Hydroxygruppen aufweisen, steht.

3. Verbindungen gemäß Anspruch 1, dadurch gekennzeichnet, daß A für Polylysin, das ein Defizit von n Aminogruppen aufweist, steht.

4. Verbindungen gemäß Anspruch 1, dadurch gekennzeichnet, daß sie Ionen der Elemente mit den Ordnungszahlen 21-29, 39, 42, 44 und 57-83 enthalten.

5. Verbindungen gemäß Anspruch 1, dadurch gekennzeichnet, daß sie Radioisotope der Elemente mit den Ordnungszahlen 27, 29, 31, 32, 37-39, 43, 49, 62, 64, 70, 75 und 77 enthalten.

6. Verwendung von mindestens einer physiologisch vertraglichen Verbindung gemäß Anspruch 4 für die Herstellung von Mitteln für die NMR- und Röntgen-Diagnostik.

7. Verwendung von mindestens einer physiologisch verträglichen Verbindung gemäß Anspruch 5 für die Herstellung von Mitteln für die Radio-Diagnostik und Radiotherapie.

8. Verbindungen gemäß Anspruch 1, dadurch gekennzeichnet, daß n für die Zahlen 1 bis 10 steht.

9. Verbindungen gemäß Anspruch 1, dadurch gekennzeichnet, daß n für die Zahlen 10 bis 200 steht.

10. Pharmazeutische Mittel enthaltend, mindestens eine physiologisch verträgliche Verbindung nach Anspruch 1, gegebenenfalls mit den in der Galenik üblichen Zusätzen.

11. Verfahren zur Herstellung von Verbindungen der allgemeinen Formel I
(M)ₙA (I),
worin
M für den Rest eines makrocyclischen Komplexbildners,
A für ein Backbone-Molekül, das ein Defizit von n Amino-, n Hydroxy- oder n Carboxygruppen aufweist,
n für die Zahlen 1 bis 400,
M unabhängig aneinander für Komplexbildner der allgemeinen Formel IA steht 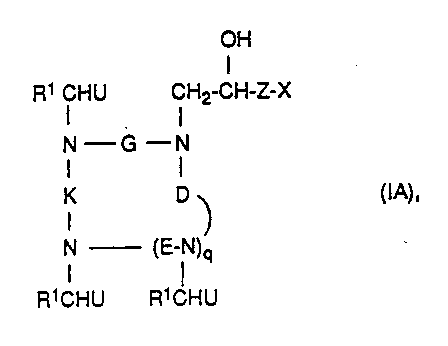 wobei
D, E, G, K, die gleich oder verschieden sein können, jeweils für die Gruppe -(CH₂)ₒ- mit o in der Bedeutung der Ziffern 2, 3, 4 oder 5
q für die Ziffern 0, 1 oder 2,
R¹ für ein Wasserstoffatom, eine verzweigte oder unverzweigte C₁-C₄-Alkyl-, Phenyl-, C₇-C₁₂-Aralkylgruppe,
U für eine CO₂H- oder PO₃H₂-Gruppe,
Z für eine gegebenenfalls Imino-, Phenylen-, Phenylenoxy-, Phenylenimino-, Amid-, Hydrazid-, Carbonyl-, Estergruppe(n), Sauerstoff-, Schwefel- und/oder Stickstoff-Atom(e) enthaltende, gegebenenfalls durch Hydroxy-, Mercapto-, Oxo-, Thioxo-, Carboxy-, Ester- und/oder Aminogruppe(n) substituierte geradkettige, verzweigte, gesättigte oder ungesättigte C₁-C₂₀-Alkylengruppe,
X für eine -CONH-, -NHCO-, -NHCSNH-, -NHCONH-, -CO₂-, -O-, -OCO-, -OCOH₂O-, -NR²CH₂CONH-, -NHCS, -NR²CH₂COO-, -OCHR²CONH-,-OCHR²COO-, wobei R² für ein Wasserstoffatom oder die Gruppe -(CH₂)_{P}COOH mit p in der Bedeutung der Ziffern 1-5 steht,und für eine -NR-gruppe, worin R ein Wasserstoffatom, einen gegebenenfalls durch 1 bis 3 Carboxy-, 1 bis 3 Sulfonsäuren, 1 bis 5 Hydroxy-, 1 bis 5 C₁-C₁₀-Alkoxy-, 1 bis 5 C₆-C₁₀-Aryloxy-, 1 bis 5 C₇-C₁₁-Aralkoxy-, 1 bis 5 Ester-, 1 bis 5 Amidgruppen substituierten und/oder gegebenenfalls 1 bis 3 Carbonyl-, 1 bis 3 Ester-, 1 bis 3 Amid-, eine Sulfonylgruppe, 1 bis 10 Sauerstoff-, 1 bis 4 Stickstoffatome enthaltenden C₁-C₂₀-Kohlenwasserstoffrest oder ein zweites Molekül IA, das mit dem ersten nicht identisch sein muß, bedeutet, stehen, deren vollständig oder unvollständig metallierte Komplexe mit den Elementen der Ordnungszahlen 21-29,31,32,37-39, 42-44,49,57-83 sowie deren Salze. dadurch gekennzeichnet, daß man in an sich bekannter Weise n Moleküle der allgemeinen Formel IA' 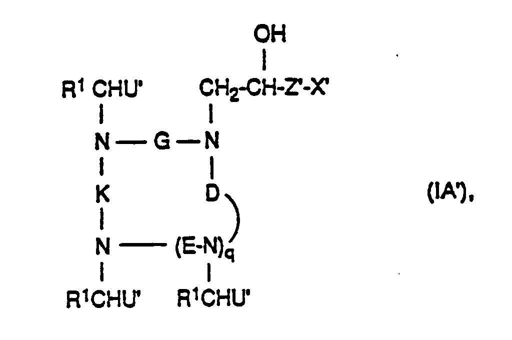 worin
Z' für Z oder eine direkte Bindung steht,
U' für eine CO₂Y oder PO₃HY-Gruppe mit Y in der Bedeutung eins Wasserstoffatoms, eines Metallionenäquivalents eines Elements der Ordnungszahlen 21-29, 31, 32, 37-39, 42-44 oder 57-83 oder einer Säureschutzgruppe,
X' für eine C*O-, NH₂-, NCS-, NCO-, QFu-, Hal-, 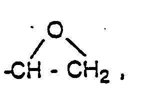 COCH₂Hal-, CHO- und Anhydridgruppe mit C*O in der Bedeutung einer aktivierten Carbonylgruppe, Fu in der Bedeutung einer Fluchtgruppe oder eines Wasserstoffatoms und Hal in der Bedeutung eines Fluor-, Chlor-, Brom- oder Jodatoms steht,
mit einem Backbone-Molekül A', das mindestens n Amino-, n Hydroxy- oder n-Carboxygruppen enthält, umsetzt,
anschließend gegebenenfalls reduziert und gewünschtenfalls mit einem den Substituenten R einführenden Reaktionspartner umsetzt, dann gegebenenfalls die Säureschutzgruppen entfernt und gegebenenfalls - in an sich bekannter Weise - mit mindestens einem Metalloxid oder Metallsalz eines Elements der Ordnungszahlen 21-29, 31, 32, 37-39, 42-44 oder 57-83 umsetzt (wobei die Metallkomplexierung auch vor der Einführung des Substituenten R erfolgen kann), und gegebenenfalls anschließend noch vorhandene acide Wasserstoffatome ganz- oder teilweise durch Kationen anorganischer und/oder organischer Basen, Aminosäuren oder Aminosäureamide substituiert.

12. Verfahren zur Herstellung der pharmazeutischen Mittel nach Anspruch 10, dadurch gekennzeichnet, daß man den in Wasser oder physiologischer Salzlösung gelösten oder suspendierten Polymerkomplex, gegebenenfalls mit den in der Galenik üblichen Zusätzen, in eine für die enterale oder parenterale Applikation geeignete Form bringt.

## Claims

1. Polymer compounds of the general formula I
(M)ₙA (I)
in which
M represents the radical of a macrocyclic complexing agent,
A represents a backbone molecule that has a deficit of n amino, n hydroxy or n carboxy groups,
n represents a number from 1 to 400, and
each M, independently of the other(s), represents a complexing agent of the general formula IA 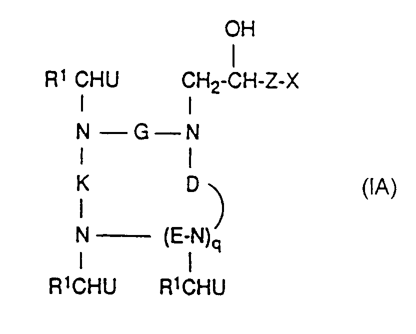 in which
D, E, G, K, which may be identical or different, each represents the group -(CH₂)ₒ-, wherein o represents the number 2, 3, 4 or 5,
q represents the number 0, 1 or 2,
R¹ represents a hydrogen atom, a branched or unbranched C₁-C₄alkyl, phenyl, C₇-C₁₂aralkyl group,
U represents a CO₂H- or PO₃H₂- group,
Z represents a straight-chained, branched, saturated or unsaturated C₁-C₂₀alkylene group that optionally contains imino, phenylene, phenyleneoxy, phenyleneimino, amide, hydrazide, carbonyl, ester group(s), oxygen, sulphur and/or nitrogen atom(s) and that is optionally substituted by hydroxy, mercapto, oxo, thioxo, carboxy, ester and/or amino group(s), and
X represents a -CONH-, -NHCO-, -NHCSNH-, -NHCONH-, -CO₂-, -O-, -OCO-, -COCH₂O-, -NR²CH₂CONH-, -NHCS-, -NR²CH₂COO-, -OCHR²CONH-, -OCHR²COO- group, in which R² represents a hydrogen atom or the group -(CH₂)ₚCOOH, wherein p represents a number from 1 to 5, or X represents an -NR-group, in which R represents a hydrogen atom, a C₁-C₂₀hydrocarbon radical that is optionally substituted by from 1 to 3 carboxy, from 1 to 3 sulphonic acids, from 1 to 5 hydroxy, from 1 to 5 C₁-C₁₀alkoxy, from 1 to 5 C₆-C₁₀aryloxy, from 1 to 5 C₇-C₁₁-aralkoxy, from 1 to 5 ester, from 1 to 5 amide groups and/or that optionally contains from 1 to 3 carbonyl, from 1 to 3 ester, from 1 to 3 amide, a sulphonyl group, from 1 to 10 oxygen, from 1 to 4 nitrogen atoms, or R represents a second molecule IA that need not be identical to the first, their completely or partially metallised complexes with the elements having atomic numbers from 21 to 29, 31, 32, from 37 to 39, from 42 to 44, 49 and from 57 to 83, and their salts.

2. Compounds according to claim 1, characterised in that A represents polypeptides, polyallylamines, poly-[N-(2-aminoethyl)]-methacrylamides and polyamine carbohydrates each of which has a deficit of n amino, n carboxy or n hydroxy groups.

3. Compounds according to claim 1, characterised in that A represents polylysine that has a deficit of n amino groups.

4. Compounds according to claim 1, characterised in that they contain ions of the elements having atomic numbers from 21 to 29, 39, 42, 44 and from 57 to 83.

5. Compounds according to claim 1, characterised in that they contain radioisotopes of the elements having atomic numbers 27, 29, 31, 32, from 37 to 39, 43, 49, 62, 64, 70, 75 and 77.

6. Use of at least one physiologically tolerable compound according to claim 4 in the preparation of compositions for NMR and X-ray diagnostics.

7. Use of at least one physiologically tolerable compound according to claim 5 in the preparation of compositions for radio-diagnostics and radiotherapy.

8. Compounds according to claim 1, characterised in that n represents a number from 1 to 10.

9. Compounds according to claim 1, characterised in that n represents a number from 10 to 200.

10. Pharmaceutical compositions comprising at least one physiologically tolerable compound according to claim 1, optionally together with additives customary in galenical pharmacy.

11. Process for the preparation of compounds of the general formula I
(M)ₙA (I)
in which
M represents the radical of a macrocyclic complexing agent,
A represents a backbone molecule that has a deficit of n amino, n hydroxy or n carboxy groups,
n represents a number from 1 to 400, and
each M, independently of the other(s), represents a complexing agent of the general formula IA 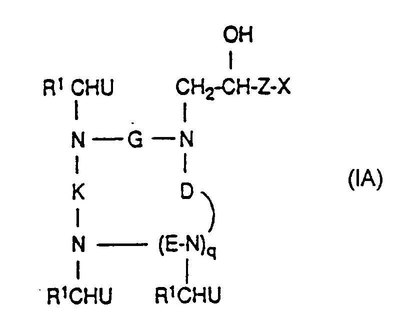 in which
D, E, G, K, which may be identical or different, each represents the group -(CH₂)ₒ-, wherein o represents the number 2, 3, 4 or 5,
q represents the number 0, 1 or 2,
R¹ represents a hydrogen atom, a branched or unbranched C₁-C₄alkyl, phenyl, C₇-C₁₂aralkyl group,
U represents a CO₂H- or PO₃H₂- group,
Z represents a straight-chained, branched, saturated or unsaturated C₁-C₂₀alkylene group that optionally contains imino, phenylene, phenyleneoxy, phenyleneimino, amide, hydrazide, carbonyl, ester group(s), oxygen, sulphur and/or nitrogen atom(s) and that is optionally substituted by hydroxy, mercapto, oxo, thioxo, carboxy, ester and/or amino group(s), and
X represents a -CONH-, -NHCO-, -NHCSNH-, -NHCONH-, -CO₂-, -O-, -OCO-, -COCH₂O-, -NR²CH₂CONH-, -NHCS-, -NR²CH₂COO-, -OCHR²CONH-, -OCHR²COO- group, in which R² represents a hydrogen atom or the group -(CH₂)ₚCOOH, wherein p represents a number from 1 to 5, or X represents an -NR-group, in which R represents a hydrogen atom, a C₁-C₂₀hydrocarbon radical that is optionally substituted by from 1 to 3 carboxy, from 1 to 3 sulphonic acids, from 1 to 5 hydroxy, from 1 to 5 C₁-C₁₀alkoxy, from 1 to 5 C₆-C₁₀aryloxy, from 1 to 5 C₇-C₁₁-aralkoxy, from 1 to 5 ester, from 1 to 5 amide groups and/or that optionally contains from 1 to 3 carbonyl, from 1 to 3 ester, from 1 to 3 amide, a sulphonyl group, from 1 to 10 oxygen, from 1 to 4 nitrogen atoms, or R represents a second molecule IA that need not be identical to the first, their completely or partially metallised complexes with the elements having atomic numbers from 21 to 29, 31, 32, from 37 to 39, from 42 to 44, 49 and from 57 to 83, and their salts,
characterised in that, in a manner known *per se*, n molecules of the general formula IA' 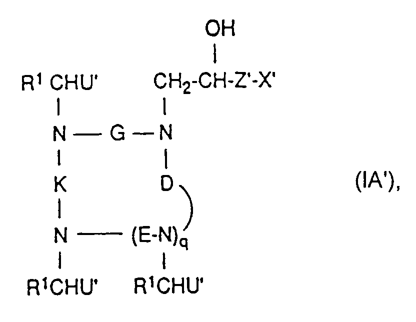 in which
Z' represents Z or a direct bond,
U' represents a CO₂Y- or PO₃HY- group wherein Y represents a hydrogen atom, a metal ion equivalent of an element having an atomic number from 21 to 29, 31, 32, from 37 to 39, from 42 to 44 or from 57 to 83, or an acid-protecting group, and
X' represents a C*O-, NH₂-, NCS-, NCO-, OFu-, Hal-, 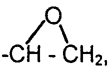 COCH₂Hal-, CHO- or anhydride group wherein C*O represents an activated carbonyl group, Fu represents a leaving group or a hydrogen atom, and Hal represents a fluorine, chlorine, bromine or iodine atom,
are reacted with a backbone molecule A' that contains at least n amino, n hydroxy or n carboxy groups,
and the resulting compound is then optionally reduced and, if desired, reacted with a reactant introducing the substituent R and then, where appropriate, the acid-protecting groups are removed and the compound is optionally reacted - in a manner known *per se* - with at least one metal oxide or metal salt of an element having an atomic number from 21 to 29, 31, 32, from 37 to 39, from 42 to 44 or from 57 to 83 (it also being possible to carry out the metal complexing before the introduction of the substituent R), and then, optionally, any acid hydrogen atoms still present are replaced wholly or partially by cations of inorganic and/or organic bases, amino acids or amino acid amides.

12. Process for the preparation of the pharmaceutical compositions according to claim 10, characterised in that the polymer complex, dissolved or suspended in water or physiological saline, optionally together with additives customary in galenical pharmacy, is brought into a form suitable for enteral or parenteral administration.

## Revendications

1. Composés polymères de formule générale I
(M)ₙA (I),
dans laquelle
M représente le reste d'un agent complexant macrocyclique,
A représente une molécule-charpente qui présente un déficit en n groupes amino, n groupes hydroxy ou n groupes carboxy,
n va de 1 à 400,
M indépendamment l'un de l'autre, représente l'agent complexant de formule générale IA 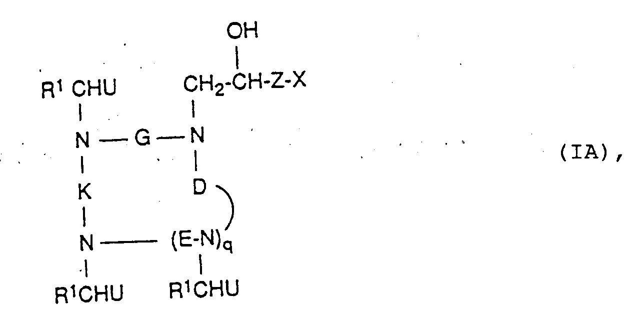 où
D, E, G, K, qui peuvent être identiques ou différents, représentent chacun le groupe -(CH₂)ₒ- avec o valant 2, 3, 4 ou 5, q vaut 0, 1 ou 2,
R¹ représente un atome d'hydrogène, un groupe alkyle en C₁-C₄ linéaire ou ramifié, phényle, aralkyle en C₇-C₁₂,
U représente un groupe CO₂H ou PO₃H₂,
Z représente un groupe alkylène en C₁-C₂₀, saturé ou insaturé, linéaire, ramifié, éventuellement contenant un groupe imino, phénylène, phénylènoxy, phénylèneimino, amido, hydrazido, carbonyle, ester, des atomes d'oxygène, de soufre et/ou d'azote, ledit groupe alkylène en C₁-C₂₀ éventuellement substitué par des groupes hydroxy, mercapto, oxo, thioxo, carboxy, ester et/ou amino,
X représente -CONH, -NHCO-, -NHCSNH-, -NHCONH-, -CO₂-, -O-, -OCO-, -COCH₂O-, -NR²CH₂CONH-, -NHCS-, -NR²CH₂COO-, -OCHR²CONH-, -OCHR²COO-, R² étant un atome d'hydrogène ou le groupe -(CH₂)ₚCOOH avec p étant un nombre allant de 1 à 5, et représente un groupe -NR- dans lequel R représente un atome d'hydrogène, un radical hydrocarboné en C₁-C₂₀ éventuellement substitué par 1 à 3 groupes carboxy, 1 à 3 groupes acide sulfonique, 1 à 5 groupes hydroxy, 1 à 5 groupes alcoxy en C₁-C₁₀, 1 à 5 groupes aryloxy en C₆-C₁₀, 1 à 5 groupes aralcoxy en C₇-C₁₁, 1 à 5 groupes ester, 1 à 5 groupes amido, et/ou éventuellement contenant 1 à 3 groupes carbonyle, 1 à 3 groupes ester, 1 à 3 groupes amido, 1 groupe sulfonyle, 1 à 10 atomes d'oxygène, 1 à 4 atomes d'azote, ou représente une deuxième molécule IA qui ne doit pas être obligatoirement identique à la première, leurs complexes complètement ou partiellement métallisés avec des éléments ayant des nombres atomiques 21-29, 31, 32, 37-39, 42-44, 49, 57-83, ainsi que leurs sels.

2. Composés selon la revendication 1, caractérisés en ce qu'ils présentent A pour des polypeptides, des polyallylamines, des poly-[N(2-aminoéthyl)]-méthacrylamides et des polyaminocarbohydrates qui présentent respectivement un déficit de n groupes amino, de n groupes carboxy ou n groupes hydroxy.

3. Composés selon la revendication 1, caractérisés en ce que A représente la polylysine, qui présente un déficit de n groupes amino.

4. Composés selon la revendication 1, caractérisés en ce qu'ils contiennent des ions des éléments ayant les nombres atomiques 21-29, 39, 42, 44 et 57-83.

5. Composés selon la revendication 1, caractérisés en ce qu'ils contiennent des radioisotopes des éléments ayant les nombres atomiques 27, 29, 31, 32, 37-39, 43, 49, 62, 64, 70, 74 et 77.

6. Utilisation d'au moins un composé physiologiquement toléré selon la revendication 4 pour la préparation d'agents pour le diagnostic par la RMN et par la radiographie aux rayons X.

7. Utilisation d'au moins un composé physiologiquement toléré selon la revendication 5 pour la préparation d'agents pour le radiodiagnostic et la radiothérapie.

8. Composés selon la revendication 1, caractérisés en ce que n va de 1 à 10.

9. Composés selon la revendication 1, caractérisés en ce que n va de 10 à 200.

10. Agents pharmaceutiques contenant au moins un composé physiologiquement toléré selon la revendication 1, éventuellement avec des adjuvants usuels en galénique.

11. Procédé pour la préparation de composés de formule générale I
(M)ₙA (I),
dans laquelle
M représente le reste d'un agent complexant macrocyclique,
A représente une molécule-charpente qui présente un déficit en n groupes amino, n groupes hydroxy ou n groupes carboxy,
n va de 1 à 400,
M indépendamment l'un de l'autre, représente l'agent complexant de formule générale IA 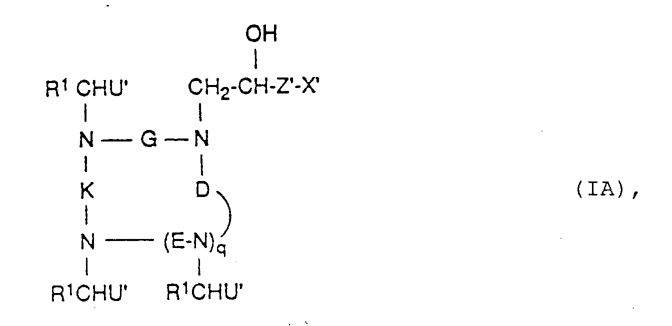 où
D, E, G, K, qui peuvent être identiques ou différents, représentent chacun le groupe -(CH₂)ₒ- avec o valant 2, 3, 4 ou 5, q vaut 0, 1 ou 2,
R¹ représente un atome d'hydrogène, un groupe alkyle en C₁-C₄ linéaire ou ramifié, phényle, aralkyle en C₇-C₁₂,
U représente un groupe CO₂H ou PO₃H₂,
Z représente un groupe alkylène en C₁-C₂₀, saturé ou insaturé, linéaire, ramifié, éventuellement contenant un groupe imino, phénylène, phénylènoxy, phénylèneimino, amido, hydrazido, carbonyle, ester, des atomes d'oxygène, de soufre et/ou d'azote, ledit groupe alkylène en C₁-C₂₀ éventuellement substitué par des groupes hydroxy, mercapto, oxo, thioxo, carboxy, ester et/ou amino,
X représente -CONH, -NHCO-, -NHCSNH-, -NHCONH-, -CO₂-, -O-, -OCO-, -COCH₂O-, -NR²CH₂CONH-, -NHCS-, -NR²CH₂COO-, -OCHR²CONH-, -OCHR²COO-, R² étant un atome d'hydrogène ou le groupe -(CH₂)ₚCOOH avec p étant un nombre allant de 1 à 5, et représente un groupe -NR- dans lequel R représente un atome d'hydrogène, un radical hydrocarboné en C₁-C₂₀ éventuellement substitué par 1 à 3 groupes carboxy, 1 à 3 groupes acide sulfonique, 1 à 5 groupes hydroxy, 1 à 5 groupes alcoxy en C₁-C₁₀, 1 à 5 groupes aryloxy en C₆-C₁₀, 1 à 5 groupes aralcoxy en C₇-C₁₁, 1 à 5 groupes ester, 1 à 5 groupes amido, et/ou éventuellement contenant 1 à 3 groupes carbonyle, 1 à 3 groupes ester, 1 à 3 groupes amido, 1 groupe sulfonyle, 1 à 10 atomes d'oxygène, 1 à 4 atomes d'azote, ou représente une deuxième molécule IA qui ne doit pas être obligatoirement identique à la première, leurs complexes complètement ou partiellement métallisés avec des éléments ayant des nombres atomiques 21-29, 31, 32, 37-39, 42-44, 49, 57-83, ainsi que leurs sels,
caractérisé en ce que l'on fait réagir de façon connue en soi n molécules de formule générale IA' 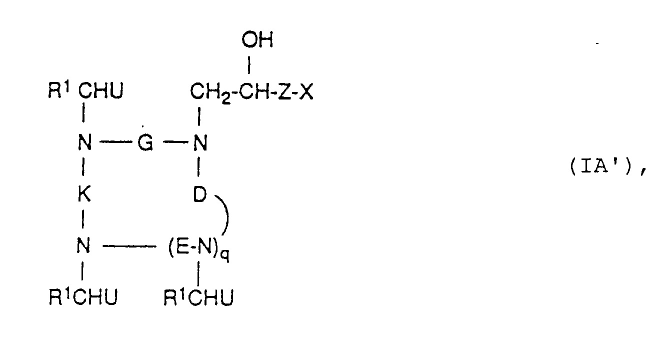 où
Z' représente Z ou une liaison directe,
U' représente un groupe CO₂Y ou PO₃HY, avec Y représentant un atome d'hydrogène, un équivalent d'un ion métallique d'un élément ayant un nombre atomique 21-29, 31, 32, 37-39, 42-44 ou 57-83 ou un groupe protecteur d'acides,
X' représente C*O-, NH₂-, NCS-, NCO-, OFu-, Hal-, 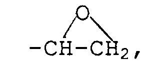 COCH₂Hal-, CHO- et un groupe anhydride avec C*O signifiant un groupe carbonyle activé, Fu signifie un groupe partant ou un groupe hydrogène et Hal signifie un atome de fluor, de chlore, de brome ou d'iode,
avec une molécule-charpente A', qui contient au moins des n groupes amino, n groupes hydroxy ou n groupes carboxy,
ensuite éventuellement on réduit et si on le souhaite, on fait réagir avec un partenaire de réaction introduisant le substituant R, puis éventuellement on élimine les groupes protecteurs d'acide et éventuellement, de façon connue en soi, on fait réagir avec au moins un oxyde métallique ou sel métallique d'un élément ayant les nombres atomiques 21-29, 31, 32, 37-39, 42-44 ou 37-83 (la complexation du métal pouvant être effectuée avant l'introduction du substituant R), et éventuellement, en substituant entièrement ou partiellement les atomes d'hydrogène acides encore présents par des cations de bases organiques et/ou inorganiques, des aminoacides ou des amide d'aminoacides.

12. Procédé de préparation d'agents pharmaceutiques selon la revendication 10, caractérisé en ce que l'on met en forme appropriée le polymère dis sous ou mis en suspension dans l'eau ou dans une solution physiologique de sel, éventuellement avec des adjuvants usuels en galénique pour des applications parentérales ou entérales.
